# EUROPEAN PATENT APPLICATION

(11) **EP 1 277 755 A1**
(43) Date of publication of application: **22.01.2003**
(21) Application number: 01921928.6
(22) Date of filing: 19.04.2001
(51) Int. Cl.: C07D 487/10, C07D 223/14, C07C 309/65, C07C 43/20, C07C 39/14, C07C 37/055, C07C 25/18, C07C 17/263, C07C 22/04, C07C 17/14, C07C 249/02, C07C 251/24, C07B 61/00, C07B 53/00, C07M 7/00

(54) **OPTICALLY ACTIVE QUATERNARY AMMONIUM SALT WITH AXIAL ASYMMETRY, PROCESS FOR PRODUCING THE SAME, AND APPLICATION TO ASYMMETRIC SYNTHESIS OF $g(a)-AMINO ACID DERIVATIVE**

(30) Priority: 21.04.2000 JP 2000121825
(71) Applicant: Nagase & Co., Ltd., Osaka-shi, Osaka 550-8668 (JP)
(72) Inventor: MARUOKA, Keiji, Shiga 520-2153 (JP)
(74) Representative: MacLean, Martin Robert
(86) International application number: JP0103373
(87) International publication number: WO01081349

(57) **Abstract**

A novel optically active quarternary ammonium salt with an axial chirality, which is useful as a phase-transfer catalyst with which a glycine derivative is stereoselectively alkylated to convert it into an optically active α-amino acid derivative, is disclosed. The quarternary ammonium salt of the present invention is, for example, a compound represented by the formula (I). Furthermore, the quarternary ammonium salt of the present invention can be used as a phase-transfer catalyst to stereoselectively alkylate a compound having a defined structure.

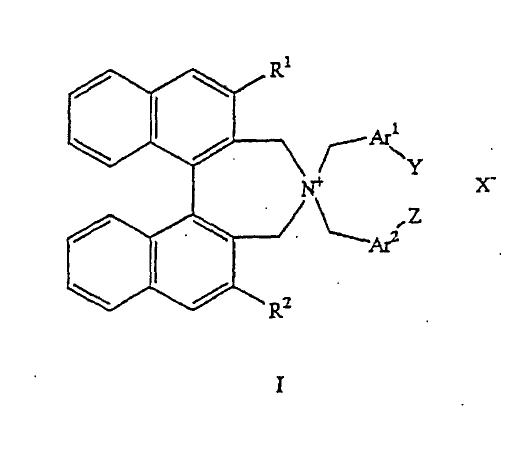

## Description

### Technical Field

The present invention relates to a novel optically active quarternary ammonium salt having C₂-symmetric axial chirality, a method for producing thereof, and an intermediate for producing the salt and a method for producing the intermediate. Furthermore, the present invention relates to a method for producing optically active α-amino acid derivatives by stereoselective alkylation using the salts as a phase-transfer catalyst.

### Background Art

A method is reported that cincona alkaloid derivatives are used as a phase-transfer catalyst for producing optically active α-amino acid derivatives represented by the formula (XXI): 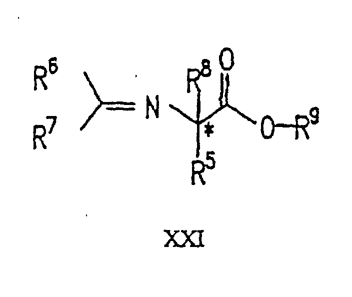 wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which having 3 to 9 carbon atoms which may be branched; and * is a newly produced chiral center; R⁶ and R⁷ are the same or different and may be a hydrogen atom, an aryl group which may be substituted with an alkyl group having 1 to 3 carbon atoms, an alkoxy group having 1 to 3 carbon atoms or a halogen atom, with the proviso that they are not both hydrogen atoms; R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, or an alkoxy group having 1 to 3 carbon atoms or a halogen atom; and R⁹ is an alkyl group having 1 to 4 carbon atoms (Corey, E.J., et al. J. Am. Chem. Soc., 1997, 119, 12414). The above-mentioned compound of the formula (XXI) are produced by stereoselectively alkylating a glycine derivative represented by the formula (XIX): 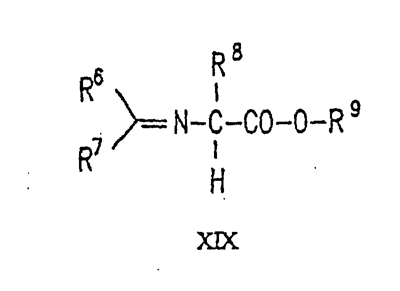 (wherein R⁶ and R⁷ are the same or different and may be a hydrogen atom, an aryl group which may be substituted with an alkyl group having 1 to 3 carbon atoms, an alkoxy group having 1 to 3 carbon atoms or a halogen atom, with the proviso that they are not both hydrogen atoms; R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, or an alkoxy group having 1 to 3 carbon atoms or a halogen atom; and R⁹ is an alkyl group having 1 to 4 carbon atoms), in the two phase system comprised of an organic solvent and water, with a compound of the formula (XX):

R⁵-W (XX)

wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 3 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which having 3 to 9 carbon atoms which may be branched; and W is a functional group having leaving ability. However, in the above-mentioned conventional method, a halogenated solvent should be used as a solvent, and since a reaction temperature should be very low for producing the chiral derivatives with high purity, so that the reaction is time-consuming, the above-mentioned method is not used for industrial production.

Furthermore, since the phase-transfer catalyst is prepared from cincona alkaloids, it is often difficult to modify derivatives of a catalyst so as to have a desired stereoselectivity based on the above-mentioned phase-transfer catalyst.

On the other hand, it is not known that an optically active quarternary ammonium salt with axial chirality is prepared and used for stereoselective alkylation as a phase-transfer catalyst.

### Disclosure of Invention

The purpose of the present invention is to provide a novel optically active quarternary ammonium salts with axial chirality, particularly their spiro derivatives, as a phase-transfer catalyst. The quarternary ammonium salt of the present invention converts glycine derivatives into optically active α-amino acid derivatives by stereoselectively alkylating the glycine derivatives. Moreover, the further purpose of the present invention is to provide an intermediate useful for production of the novel quarternary ammonium salt.

The present invention provides a compound of the formula (Ia): 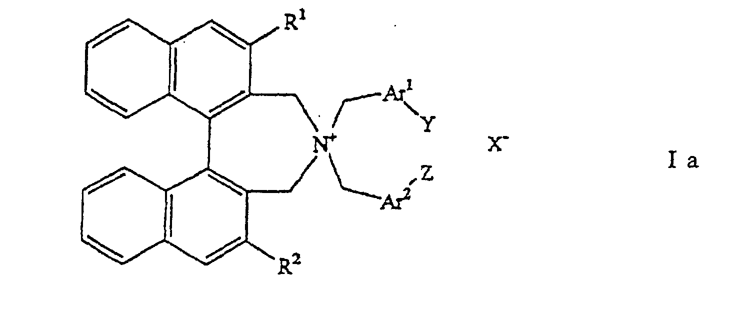 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different;
X⁻ is a halide anion; and
Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond,
with the proviso that the case where both R¹ and R² are a hydrogen atom and both Ar¹-Y and Ar²-Z are an unsubstituted phenyl group or an unsubstituted α-naphthyl group, and the case where both R¹ and R² are a hydrogen atom, an unsubstituted phenyl group or an unsubstituted β-naphthyl group and Ar¹-Y and Ar²-Z form a group represented by the following formula: 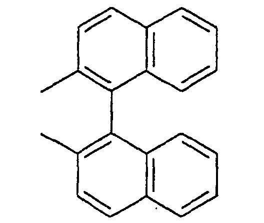 are excluded. Thus, the above purposes are achieved.

The description "which may be branched or form a cyclic group", used herein, means "which may be a linear chain, branched chain or ring structure".

The above compound is a spiro type and Y and Z form a single bond, and the compound is represented by the formula (IIa):

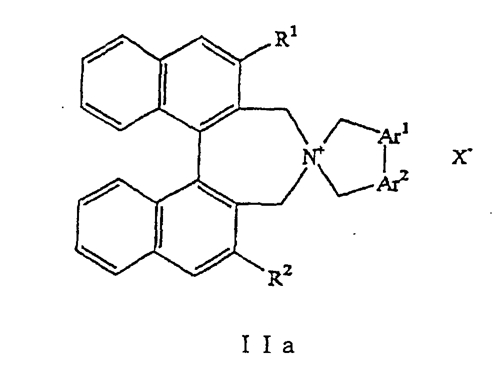

Moreover, the present invention provides a method for producing the compound of the formula (Ia), comprising reacting a compound of the formula (IVa): 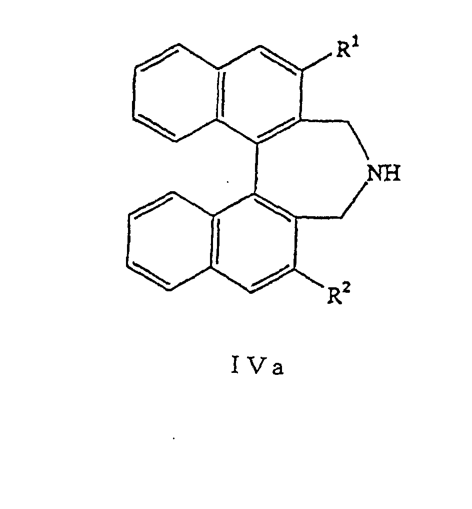 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different, with the proviso that the case where both R¹ and R² are a hydrogen atom is excluded; with a compound of the formula (V): 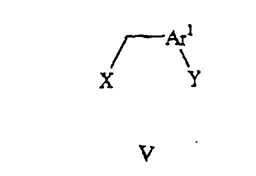 and a compound of the formula (VI): 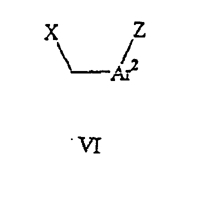 wherein, in the formulae (V) and (VI), Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, Ar¹ and Ar² may be the same or different;
X is a halogen atom; and
Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond;
in this order or simultaneously, in the presence of an acid scavenging agent and in an appropriate solvent.

Moreover, the present invention provides a method for producing the compound of the formula (IIa), comprising reacting a compound of the formula (IVa): 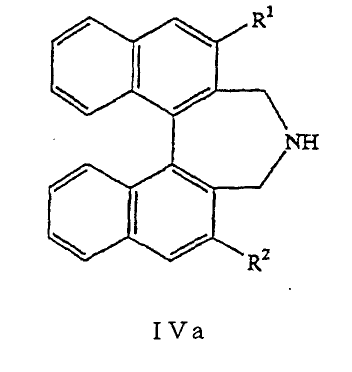 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
with a compound of the formula (VIIa): 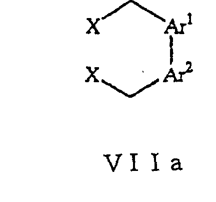 wherein Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different; X is a halogen atom; with the proviso that the case where both R¹ and R² are a hydrogen atom and the case where 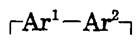 is a binaphthyl group which is bound to an unsubstituted phenyl group or an unsubstituted α-naphthyl group are excluded; in the presence of an acid scavenging agent and in an appropriate solvent.

Moreover, the present invention provides a compound of the formula (VIIIa): 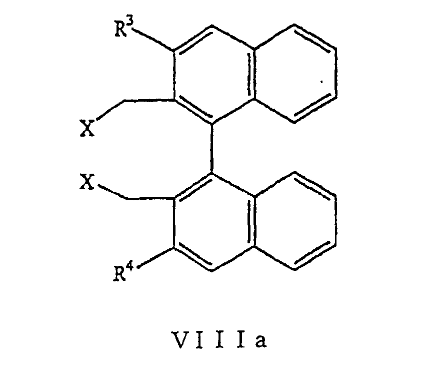 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R³ and R⁴ may be the same or different; X is a halogen atom, with the proviso that the case where both R³ and R⁴ are a hydrogen atom is excluded.

In a preferred embodiment of the present invention, X may be a bromine atom.

Moreover, the present invention provides a compound of the formula (XIa): 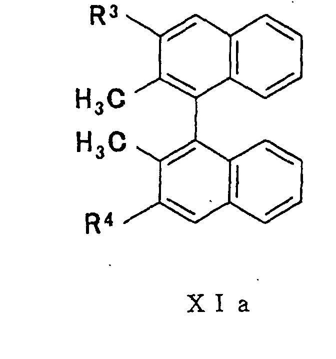 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R³ and R⁴ may be the same or different, with the proviso that the case where both R³ and R⁴ are a hydrogen atom is excluded.

Further, the present invention provides a method for producing the compound of the formula (VIIIa), comprising reacting the compound of the formula (XIa) with an appropriate halogenating agent which can generate a halogen radical in an appropriate solvent and in the presence of a radical reaction initiator to halogenate both 2- and 2'-methyl groups.

Moreover, the present invention provides a compound of the formula (XIIa): 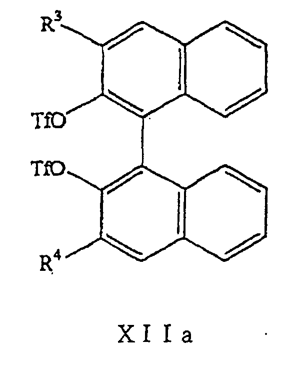 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₃ alkyl) carbamoyl group; and a N,N-di(C₁ to C₃ alkyl) carbamoyl group (wherein alkyl may be the same or different), and R³ and R⁴ may be the same or different; and Tf is a trifluoromethanesulfonyl group, with the proviso that the case where both R³ and R⁴ are an unsubstituted phenyl group or an unsubstituted β-naphthyl group is excluded.

Moreover, the present invention provides a method for producing the compound of the formula (XIa), comprising reacting the compound of the formula (XIIa) with a methylmagnesium halide represented by a formula (XIII):

MeMgX (XIII)

wherein X is a halogen atom,
in the presence of a nickel catalyst in an appropriate solvent.

Moreover, the present invention provides a method for producing a compound of the formula (XIIa'): 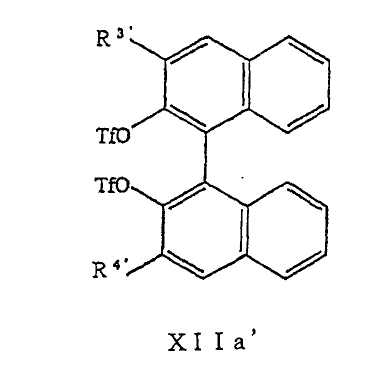 wherein R^{3'} and R^{4'} are groups independently selected from the group consisting of an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₃ alkyl) carbamoyl group; and a N,N-di(C₁ to C₃ alkyl) carbamoyl group (wherein alkyl may be the same or different), and R^{3'} and R⁴' may be the same or different; and Tf is a trifluoromethanesulfonyl group, with the proviso that the case where both R^{3'} and R^{4'} are an unsubstituted phenyl group or an unsubstituted β-naphthyl group is excluded, comprising:
substituting a bromine atom in the compound of the formula (XIV): 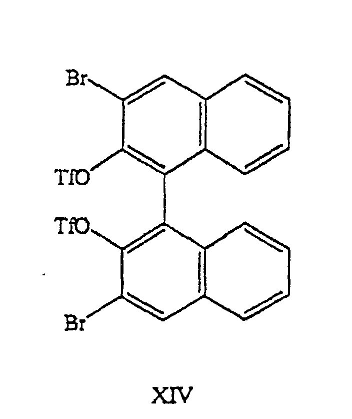 wherein Tf is a trifluoromethanesulfonyl group, with R^{3'} and R^{4'} in an appropriate solvent and in the presence of a transition metal catalyst.

Moreover, the present invention provides a method for producing the compound of the formula (XVIIa): 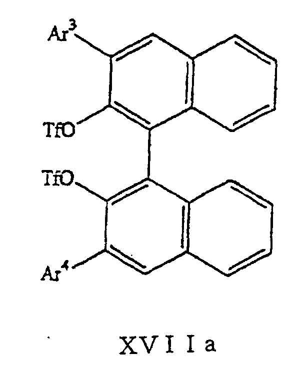 wherein Ar³ and Ar⁴ are groups selected from the group consisting of an alkenyl group having 2 to 6 carbon atoms which may be branched or form a cyclic group; an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar³ and Ar⁴ may be the same or different, with the proviso that the case where both Ar³ and Ar⁴ are an unsubstituted phenyl group or an unsubstituted β-naphthyl group is excluded; comprising
reacting the compound of the formula (XIV): 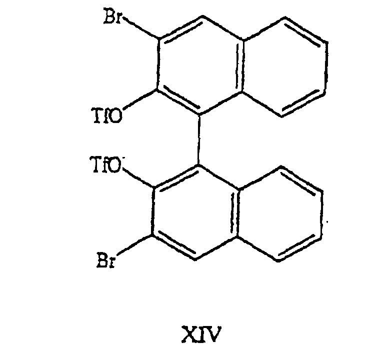 wherein Tf is a trifluoromethanesulfonyl group, with a compound of the formula (XV):

Ar³B(OH)₂ (XV)

and a compound of the formula (XVI):

Ar⁴B(OH)₂ (XVI)

wherein, in the formulae (XV) and (XVI), Ar³ and Ar⁴ are groups selected from the group consisting of an alkenyl group having 2 to 6 carbon atoms which may be branched or form a cyclic group; an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms, or a halogen atom, and Ar³ and Ar⁴ may be the same or different, with the proviso that the case where both Ar³ and Ar⁴ are an unsubstituted phenyl group or an unsubstituted β-naphthyl group is excluded;
in this order or simultaneously, in an appropriate solvent and in the presence of a base and a palladium catalyst.

Moreover, the present invention provides a compound of the formula (XVIII):

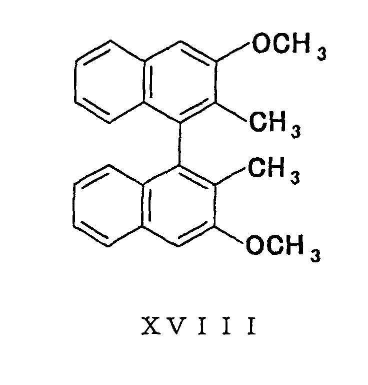

Moreover, the present invention provides a method for producing the compound of the formula (XVIII): 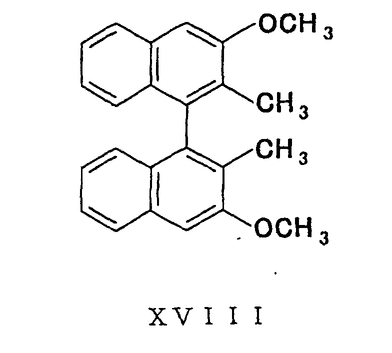 comprising:
sulfonating a compound of the formula (XXI): 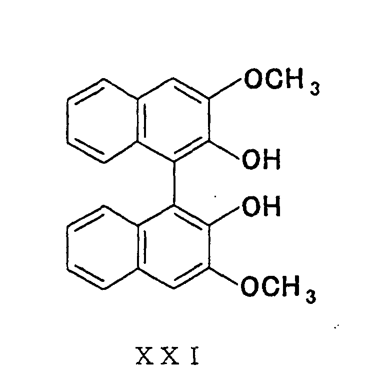 to obtain a compound of the formula (XXII): 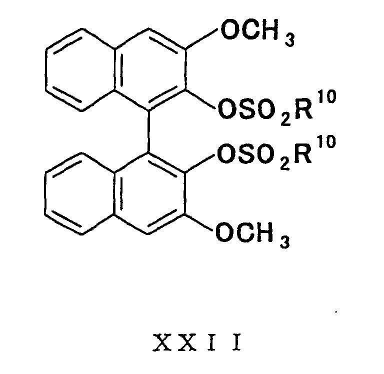 wherein R¹⁰ is an alkyl group having 1 to 4 carbon atoms which may be substituted with a halogen atom; and
reacting the compound of the formula (XXII) with a methylmagnesium halide of the formula (XIII):

MeMgX (XIII)

wherein X is a halogen atom, in an appropriate solvent and in the presence of a nickel catalyst.

Moreover, the present invention provides a compound of the formula (XXIII):

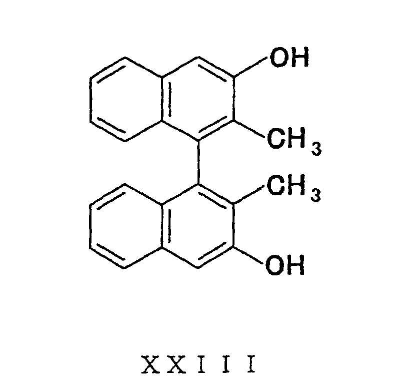

Moreover, the present invention provides a method for producing the compound of the formula (XXIII): 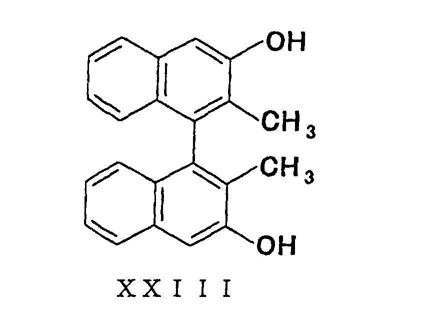 comprising:
reacting the compound of the formula (XVIII): 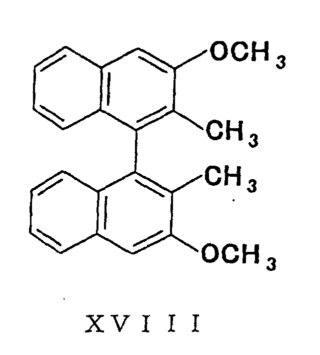 with boron tribromide.

Moreover, the present invention provides a compound of the formula (XXIV):

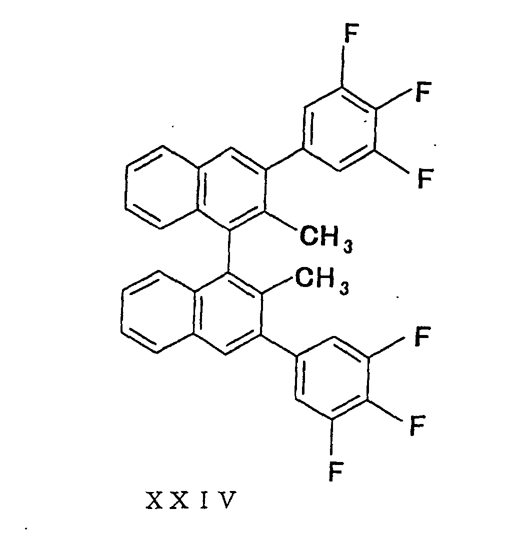

Moreover, the present invention provides a method for producing the compound of the formula (XXIV): 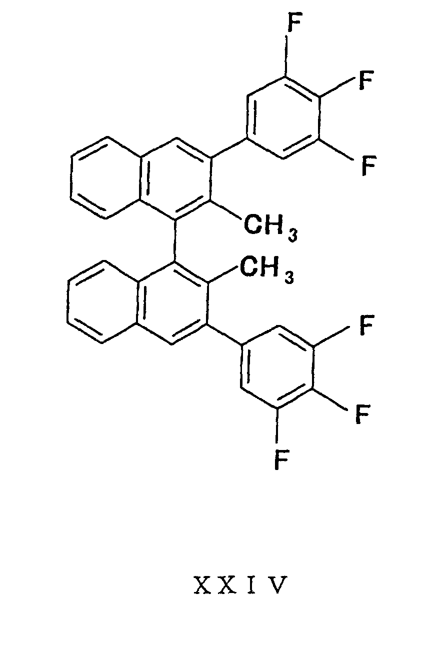 comprising:
sulfonating a compound of the formula (XXIII): 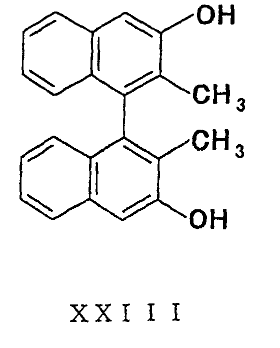 to generate a compound of the formula (XXV): 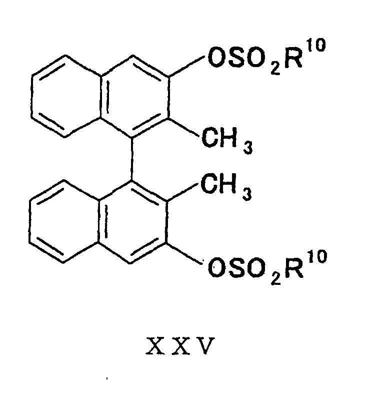 wherein R¹⁰ is an alkyl group having 1 to 4 carbon atoms which may be substituted with a halogen atom; and
reacting the compound of the formula (XXV) with 3,4,5-trifluorophenyl boronic acid, in an appropriate solvent and in the presence of a palladium catalyst.

Moreover, the present invention provides a compound of the formula (XXVI):

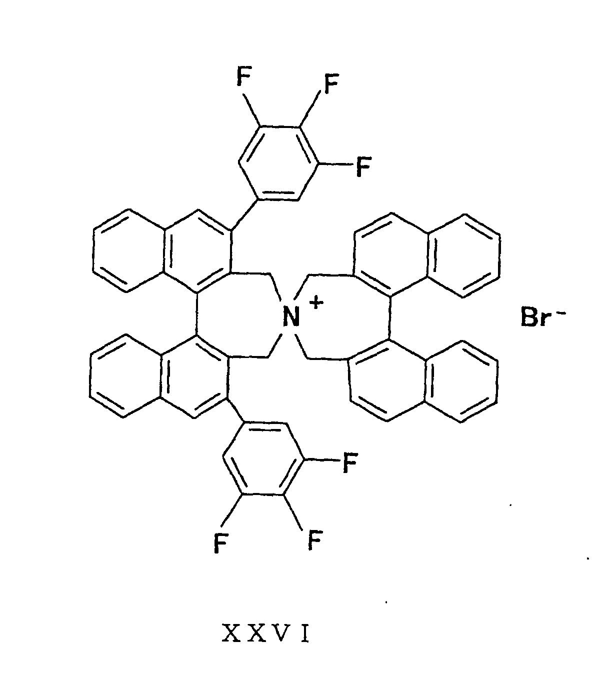

Moreover, the present invention provides a method for producing the compound of the formula (XXVI): 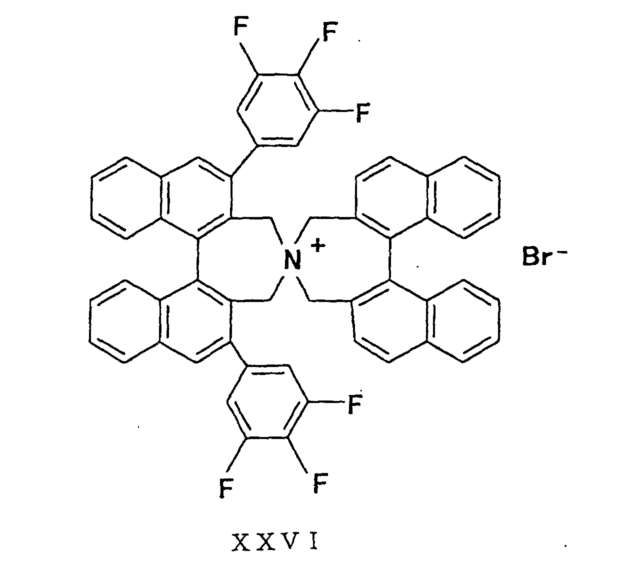 comprising:
reacting a compound of the formula (XXIV): 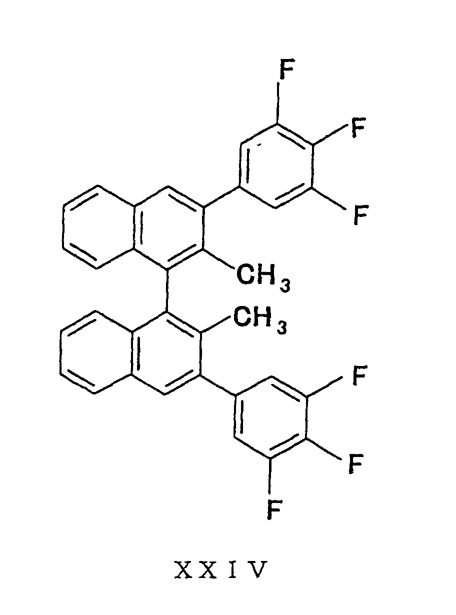 with a brominating agent, for example, bromosuccinimide, and 2,2'-azobis(isobutylonitrile), in an appropriate solvent to generate a compound of the formula (XXVII): 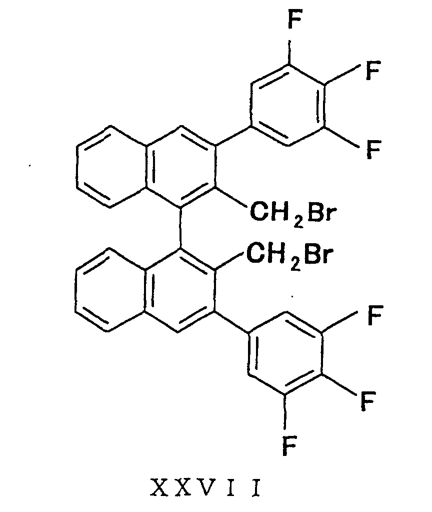 and
reacting the compound of the formula (XXVII) with a compound of the formula (XXVIII):

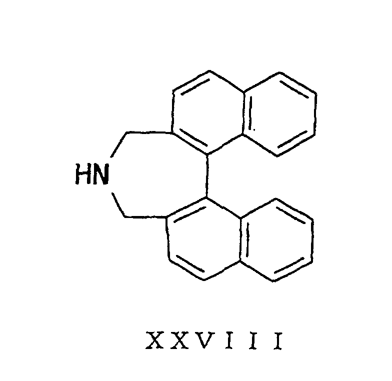

Further, the present invention provides a method for stereoselectively producing a compound of the formula (XXIa): 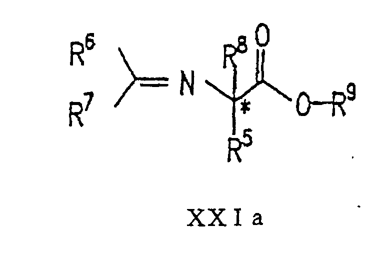 wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which has 3 to 9 carbon atoms which may be branched;
R⁶ and R⁷ are the same or different and may be a hydrogen atom, an aryl group which may be substituted with an alkyl group having 1 to 3 carbon atoms, an alkoxy group having 1 to 3 carbon atoms or a halogen atom, with the proviso that they are not both hydrogen atoms;
R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, or an alkoxy group having 1 to 3 carbon atoms or a halogen atom;
R⁹ is an alkyl group having 1 to 4 carbon atoms; and
* is a newly produced chiral center,
comprising;
alkylating a compound represented by the formula (XIXa): 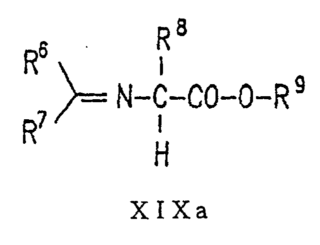 wherein R⁶ and R⁷ are the same or different and may be a hydrogen atom, an aryl group which may be substituted with an alkyl group having 1 to 3 carbon atoms, an alkoxy group having 1 to 3 carbon atoms or a halogen atom, with the proviso that they are not both hydrogen atoms; R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, an alkoxy group having 1 to 3 carbon atoms or a halogen atom; and R⁹ is an alkyl group having 1 to 4 carbon atoms;
with a compound of the formula (XX):

R⁵-W (XX)

wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 3 carbon atoms, an alkoxy group having 1 to 3 carbon atoms, a halogen atom, an aryl group which may be substituted with an alkyl group having 1 to 3 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which has 3 to 9 carbon atoms which may be branched; and W is a functional group having leaving ability;
using the compound of the formula (I): 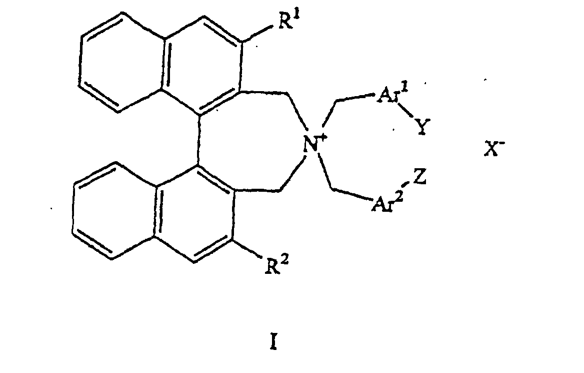 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different;
X⁻ is a halide anion; and
Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond;
which is pure regarding axial chirality, as a phase-transfer catalyst in an appropriate solvent and in the presence of an inorganic base, with the proviso that the case where, in the compound of the formula (I), both R¹ and R² are a hydrogen atom and both Ar¹-Y and Ar²-Z are an unsubstituted phenyl group or an unsubstituted α-naphthyl group, and, in the compound of the formula (XX), R⁵ is a benzyl group; the case where, in the compound of the formula (I), both R¹ and R² are an unsubstituted β-naphthyl group and Ar¹-Y and Ar²-Z form a group represented by the following formula: 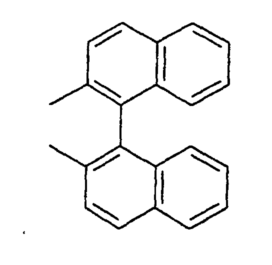 and, in the compound of the formula (XX), R⁵ is a benzyl group; and the case where, in the compound of the formula (I), both R¹ and R² are an unsubstituted β-naphthyl group and, in the compound of the formula (XX), R⁵ is a benzyl group, a methyl group, an ethyl group, an allyl group, a 2-methylpropene group, a propargyl group, a p-methylbenzyl group, a p-fluorobenzyl group, or a group represented by the following formula: 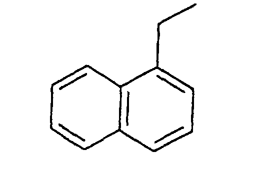 are excluded.

In the above method, as the phase-transfer catalyst, the compound of the following formula (II) may be used: 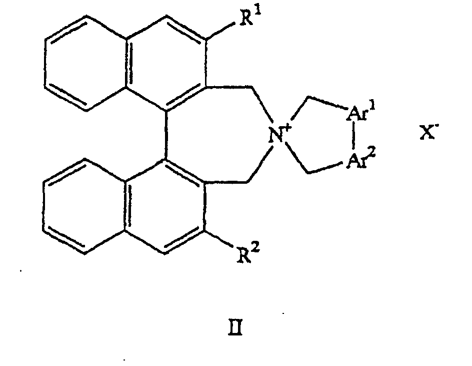 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different;
X⁻ is a halide anion; and
Y and Z are groups independently selected from the group consisting of a hydrogen atom; a halogen atom; an alkyl group having 1 to 4 carbon atoms; and an alkoxy group having 1 to 3 carbon atoms;
with the proviso that the case where both R¹ and R² are a hydrogen atom, an unsubstituted phenyl group, or an unsubstituted β-naphthyl group, and both Ar¹-Y and Ar²-Z form a group represented by the following formula: 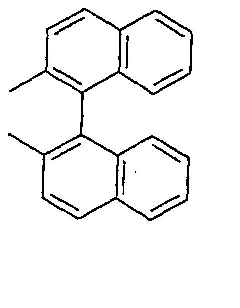 is excluded.

In the above method, as the phase-transfer catalyst, the compound of the following formula (III) may be used: 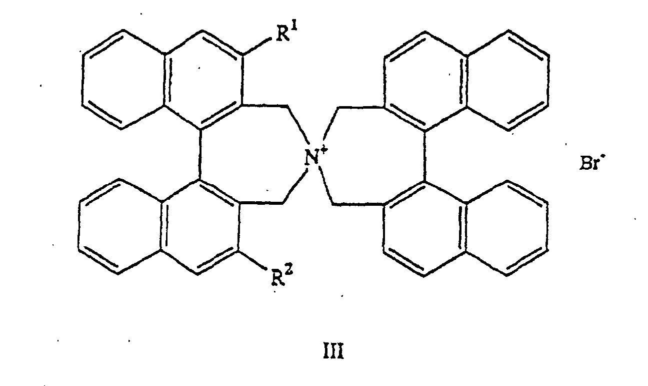 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
with the proviso that the case where both R¹ and R² are a hydrogen atom, an unsubstituted phenyl group, or an unsubstituted β -naphthyl group is excluded.

In the above method, as the phase-transfer catalyst, the compound of the following formula (III) wherein both R¹ and R² is the following formula may be used:

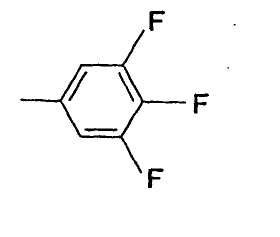

The phrases and terms used herein are defined as follows:

The term "alkyl group(s) having 1 to 6 carbon atoms which may be branched or form a cyclic group" means any of linear, branched or cyclic alkyl group(s) having 1 to 6 carbon atoms, including a methyl, ethyl, n-propyl, isopropyl, cyclopropyl, n-butyl, sec-butyl, tert-butyl, cyclobutyl, pentyl, cyclopentyl, hexyl and cyclohexyl group. In the present invention, a methyl, isopropyl or tert-butyl group is preferable.

The term "alkenyl group(s) having 2 to 6 carbon atoms which may be branched or form a cyclic group" means any of linear, branched or cyclic alkenyl group(s) having 2 to 6 carbon atoms, including an ethenyl, propenyl, isopropenyl, cyclopropenyl, butenyl, 1-methyl-1-propenyl, 1-methyl-2-propenyl, 2-methyl-1-propenyl, 2-methyl-2-propenyl, cyclobutenyl, pentenyl, cyclopentenyl, hexenyl and cyclohexenyl group. In the present invention, a propenyl or butenyl group is preferable.

The term "alkynyl group(s) having 2 to 6 carbon atoms and which may be branched or form a cyclic group" means any of linear, branched or cyclic alkynyl group(s) having 2 to 6 carbon atoms, including an ethynyl, propynyl, cyclopropylethynyl, butynyl, 1-methyl-2-propynyl, pentynyl, cyclobutylethynyl, hexynyl, and trimethylsilylethynyl group. In the present invention, an ethynyl or trimethylsilylethynyl group is preferable.

The term "allyl or substituted allyl group(s) having 3 to 9 carbon atoms which may be branched or form a cyclic group" means allyl group(s), or any of substituted allyl group(s) having a substituent in 1 and/or 2 and/or 3 position and having total carbon atoms of 4 to 9, including a 2-butenyl, 1-cyclopentenylmethyl, and 3-methyl-2-butenyl group. In the present invention, an allyl group is preferable.

The term "propargyl or substituted propargyl group(s) which having 3 to 9 carbon atoms which may be branched" means propargyl group, or any of substituted propargyl group(s) having substituent in 1 and/or 3 position and having 4 to 9 of total carbon atoms. The examples include 2-butynyl and 3-trimethylsilyl-2-propynyl group. In the present invention, a propargyl and 3-trimethylsilyl-2-propynyl group are preferred.

The term "a functional group having leaving ability" means an atom or an atomic group which is eliminated from a reaction substrate, in other words, a leaving group, in a substitution reaction or an elimination reaction. Examples of the group include a halogen atom and a sulfonyloxy group.

The "aralkyl group" used in the present invention includes a benzyl, phenethyl, naphthylmethyl and anthracenylmethyl group.

The "heteroaralkyl group" used in the present invention includes a pyridylmethyl, quinonylmethyl, indolylmethyl, furylmethyl, thienylmethyl and pyrolylmethyl group.

The "aryl group" used in the present invention includes a phenyl, biphenyl, naphthyl and anthracenyl group.

The "heteroaryl group" used in the present invention includes a pyridyl, quinolyl, pyrrolyl, imidazolyl, furyl, indolyl, thienyl, oxazolyl, and thiazolyl group.

The "halogen atom" used in the present invention includes a chlorine, bromine, iodine, and fluorine atom.

The "sulfonyloxy group" used in the present invention includes a methanesulfonyloxy, p-toluenesulfonyloxy and trifluoromethane sulfonyloxy group.

An appropriate solvent includes benzene, toluene, xylene, ethylether, isopropylether, tetrahydrofuran, dioxane, or the like. Furthermore, the solvent also includes two-phase system comprising water immiscible solvent and water. The inorganic base includes lithium hydroxide, sodium hydroxide, potassium hydroxide, calcium hydroxide, rubidium hydroxide, cesium hydroxide or the like.

### Best Mode for Carrying Out the Invention

Hereinafter, the present invention will be described in detail.

The inventor has found that optically active quarternary ammonium salts represented by the formula (I) that can form a spiro structure and an axial chirality, functions as an excellent phase-transfer catalyst in the two-phase medium including an organic solvent and water: 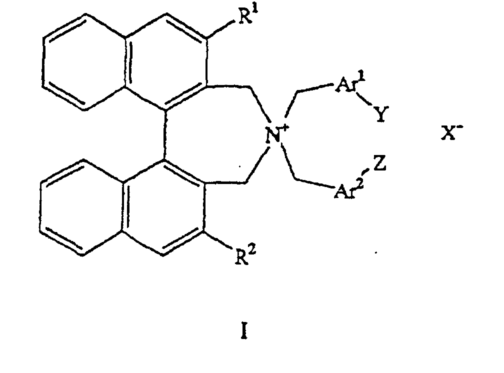 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different; Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different; X⁻ is a halide anion; and Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond. Furthermore, the inventor has found that, by using the optically active quarternary ammonium salt represented by the formula (I), an optically active α-amino acid derivative represented by the formula (XXI) having high optical purity is produced: 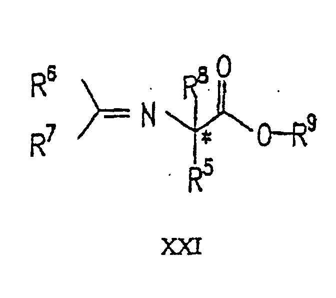 wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which has 3 to 9 carbon atoms that may be branched; R⁶ and R⁷ are the same or different and are hydrogen atoms, an aryl group which may be substituted with an alkyl group having 1 to 3 carbon atoms, an alkoxy group having 1 to 3 carbon atoms or a halogen atom, with the proviso that they are not both hydrogen atoms;
R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, an alkoxy group having 1 to 3 carbon atoms or a halogen atom; R⁹ is an alkyl group having 1 to 4 carbon atoms; and * is a newly produced chiral center;
by stereoselectively alkylating a glycine derivative represented by the formula (XIX): 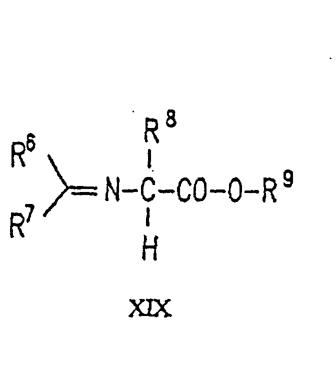 wherein R⁶ and R⁷ are the same or different and are a hydrogen atom, an aryl group which may be substituted with an alkyl group having 1 to 3 carbon atoms, an alkoxy group having 1 to 3 carbon atoms or a halogen atom, with proviso that they are not both hydrogen atoms; R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, an alkoxy group having 1 to 3 carbon atoms or a halogen atom; and R⁹ is an alkyl group having 1 to 4 carbon atoms;
with a compound represented by the formula (XX):

R⁵-W (XX)

wherein R⁶ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which has 3 to 9 carbon atoms that may be branched; and W is a functional group having an ability to eliminate a halogen atom, sulfonyloxy group and the like.

In the optically active quarternary ammonium salt with the axial chirality represented by the formula (I), when the spiro type quarternary ammonium salts with the axial chirality represented by the formula (II) are employed as a phase-transfer catalyst, a higher stereoselective alkylation can be performed: 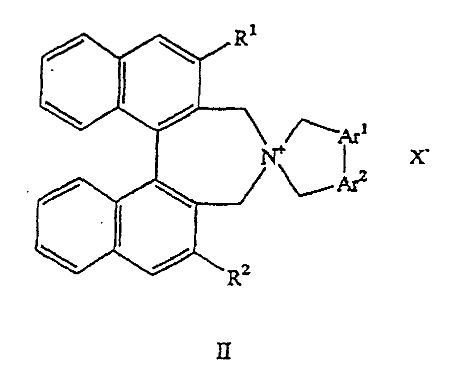 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different; and
X⁻ is a halide anion.

Particularly, in the compound of the formula (II), the optically active spiro type quarternary ammonium salt (in which Ar¹ and Ar² are β-naphthyl groups, each Ar¹ and Ar² is bound to α-position of the other group, and X⁻ is a bromide anion) having C₂-symmetric axial chirality represented by the formula (III): 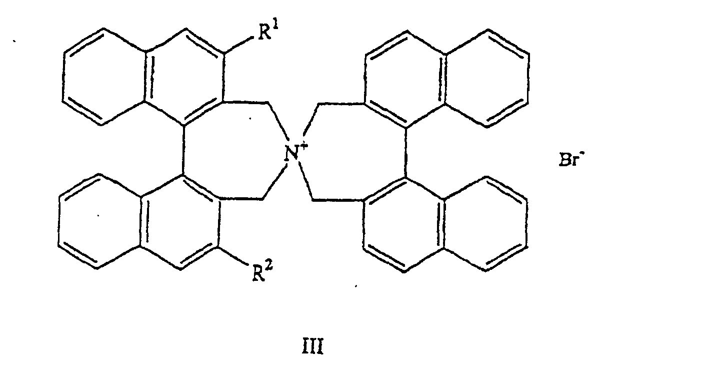 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different); R¹ and R² may be the same or different; and, in particular, in the compound of the formula (III), both R¹ and R² are a phenyl, or both R¹ and R² are a β-naphthyl;
is used as a particularly useful phase-transfer catalyst, thereby providing the above-mentioned stereoselective alkylation in 90%e.e. or more.

The optically active quarternary ammonium salt, with an axial chirality and which may form a spiro structure, represented by the formula (I) can be produced by reacting an optically active dinaphthoazepine derivative with an axial chirality, represented by the formula (IV): 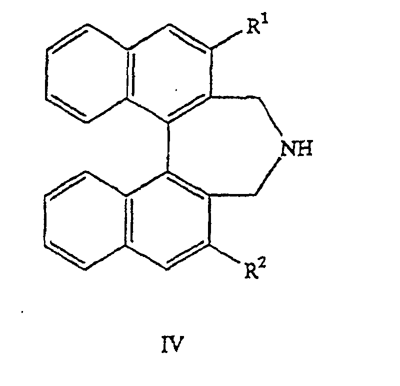 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
with a compound of the formula (V): 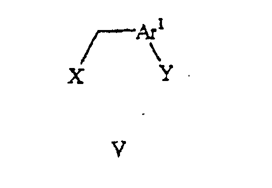 and a compound of the formula (VI): 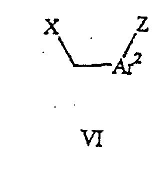 wherein, in the formulae (V) and (VI), Ar¹ and Ar² are groups independently selected from the group consisting of an aryl group (such as phenyl, biphenyl, naphthyl) which may be substituted with an alkyl group having 1 to 4 carbon atoms, an alkoxy group having 1 to 3 carbon atoms or a halogen atom; and a heteroaryl group (such as pyridyl, quinonyl) which may be substituted with an alkyl group having 1 to 4 carbon atoms, an alkoxy group having 1 to 3 carbon atoms or a halogen atom, and Ar¹ and Ar² may be the same or different;
X is a halogen atom; and
Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond;
in this order or simultaneously, in the presence of an acid scavenging agent and in an appropriate solvent.

The compound of the formula (IV) can be produced by subjecting the compound of the formula (Villa) to a method of Hawkins, J. M. et al. (Hawkins, J. M. et al., J. Org. Chem. 1994, 59, 649). A number of the compounds represented by the formulae (V) and (VI) are commercially available as a reagent. Alternatively, the compound of the formula (Villa) may be used in place of these compounds.

According to the present invention, the compound of the formula (I) can be produced by stirring a compound of the formula (IV) and compounds of the formulae (V) and (VI) in an alcoholic solvent in the presence of an acid scavenging agent at an appropriate temperature. Compounds of the formulae (V) and (VI) can be used preferably in 0.8 to 1.5 equivalents, more preferably 1.0 to 1.4 equivalents and most preferably 1.1 to 1.2 equivalents to the compound of the formula (IV), respectively. Examples of the alcohol include methanol, ethanol, propanol, isopropyl alcohol, butanol and tert-butyl alcohol. Examples of the acid scavenging agent include potassium carbonate and sodium carbonate. The temperature can be between room temperature and a boiling temperature of the solvent used, preferably between room temperature and 80°C. The stirring period can be preferably 30 minutes to 12 hours, more preferably 2 to 11 hours and most preferably 3 to 10 hours. In the reaction, the above-mentioned solvent may be used in volume (mL) preferably 5 to 50 times, more preferably 10 to 40 times the weight (g) of the compound of the formula (IV). The acid scavenging agent may be used preferably in 2 to 4 equivalents and more preferably 2 to 3 equivalents to the compound of formula (IV).

The optically active spiro type quarternary ammonium salt with an axial chirality and represented by the formula (II) can be produced by reacting a compound of the formula (IV) with a compound of the formula (VII): 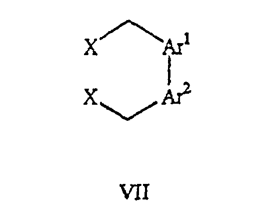 wherein Ar¹ and Ar² are groups independently selected from the group consisting of an aryl group such as a phenyl, biphenyl, naphthyl group which may be substituted with an alkyl group having 1 to 4 carbon atoms, an alkoxy group having 1 to 3 carbon atoms or a halogen atom; and a heteroaryl group such as a pyridyl group or a quinonyl group which may be substituted with an alkyl group having 1 to 4 carbon atoms, an alkoxy group having 1 to 3 carbon atoms or a halogen atom;
Ar¹ and Ar² may be the same or different; and
X is a halogen atom;
in the presence of an acid scavenging agent in an appropriate solvent.

According to the present invention, the compound of the formula (II) can be produced by stirring compounds of the formulae (IV) and (VII) in an alcohol solvent in the presence of an acid scavenging agent at an appropriate temperature. The compound of the formula (VII) can be used preferably in 1 to 3 equivalents, more preferably 1 to 2 equivalents and most preferably 1 to 1.5 equivalents to the compound of the formula (IV). Examples of the alcohol include methanol, ethanol, propanol, isopropyl alcohol, butanol and tert-butyl alcohol. Examples of the acid scavenging agent include potassium carbonate and sodium carbonate. The temperature can be between room temperature and a boiling point of the solvent used, preferably between room temperature and 80°C. The stirring period can be preferably 30 minutes to 12 hours, more preferably 1 to 11 hours and most preferably 2 to 10 hours. In the reaction, the above-mentioned solvent may be used in volume (mL) preferably 5 to 50 times, more preferably 5 to 30 times and most preferably 10 to 25 times the weight (g) of the compound of the formula (IV). The acid scavenging agent may be used preferably in 2 to 4 equivalents and more preferably 2 to 3 equivalents to the compound of the formula (IV).

The optically active spiro type quarternary ammonium salt with an axial chirality and represented by the formula (III) can be produced by reacting optically active 3,5-dihydro-4H-dinaphtho[2,1-c:1',2'-e]azepine (IX) with optically active 1,1'-binaphthyl derivative represented by the formula (X): 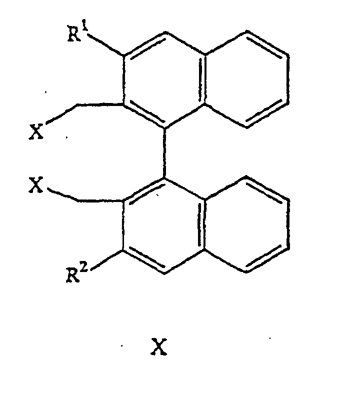 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different; and X is a halogen atom;
in alcoholic solvent in the presence of an inorganic base as an acid scavenging agent.

The optically active 3,5-diydro-4H-dinaphtho[2,1-c:1',2'-e)azepine (IX) can be produced by the method by Hawkins et al. (Hawkins, J. M. et al., J. Org. Chem. 1994, 59, 649).

According to the present invention, the compound of the formula (III) can be produced by stirring the compound (IX) with the compound of the formula (X) in an alcoholic solvent in the presence of an acid scavenging agent at an appropriate temperature. The compound of the formula (X) can be used preferably in 1 to 3 equivalents, more preferably 1 to 2 equivalents and most preferably 1 to 1.5 equivalents to the compound (IX). Examples of the alcohol include methanol, ethanol, propanol, isopropyl alcohol, butanol and tert-butyl alcohol. Examples of the acid scavenging agent include potassium carbonate and sodium carbonate. The temperature can be between room temperature and a boiling point of the solvent used, preferably between room temperature and 80°C. The stirring period can be preferably 30 minutes to 12 hours, more preferably 1 to 11 hours and most preferably 2 to 10 hours. In the reaction, the above-mentioned solvent may be used in volume (mL) preferably 5 to 50 times, and more preferably 5 to 30 times the weight (g) of the compound (IX). The acid scavenging agent may be used preferably in 2 to 4 equivalents and more preferably 2 to 3 equivalents to the compound (IX).

The compound represented by the formula (X) can be produced by reacting an optically active 2,2'-dimethyl-1,1'-binaphthyl derivative represented by the formula (XI): 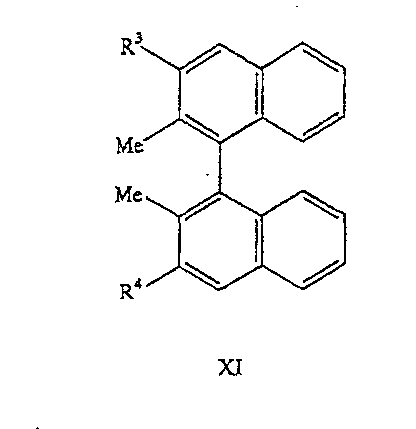 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R³ and R⁴ may be the same or different;
with an appropriate halogenating agent (halogen radical generating agent) which can generate a halogen radical in an appropriate solvent and in the presence of an appropriate radical reaction initiator to halogenate both 2- and 2'-methyl groups.

According to the present invention, the compound of the formula (X) can be produced by stirring the compound of the formula (XI) with a halogen radical generating agent in the presence of a radical reaction initiator in a hydrocarbon solvent at an appropriate temperature. The halogen radical generating agent can be used preferably in 2 to 3 equivalents and more preferably 2 to 2.5 equivalents to the compound of the formula (XI). Examples of the halogen radical generating agent include N-bromosuccinimide. Examples of the radical reaction initiator include benzoyl peroxide. Examples of the hydrocarbon solvent include hexane, cyclohexane and petroleum ether. The stirring temperature is between room temperature and a boiling point of the solvent used, preferably between 60°C and 100°C. The stirring period can be preferably 30 minutes to 5 hours, more preferably 1 to 5 hours and most preferably 1 to 3.5 hours. In the reaction, the solvent may be used in volume (mL) preferably 5 to 20 times, more preferably 5 to 15 times and most preferably 5 to 10 times the weight (g) of the compound of the formula (XI). The radical reaction initiator may be used preferably in 0.1 to 0.6 equivalents and more preferably 0.2 to 0.6 equivalents to the compound of the formula (XI). The halogen radical generating agent may be used preferably in 1 to 5 equivalents, more preferably 1.5 to 3.5 equivalents and most preferably 1.8 to 2.6 equivalents to the compound of the formula (XI).

A compound represented by the formula (XI) can be produced by reacting an optically active 2,2'-bistrifluoromethanesulfonyloxy-1,1'-binaphthyl derivative represented by the formula (XII): 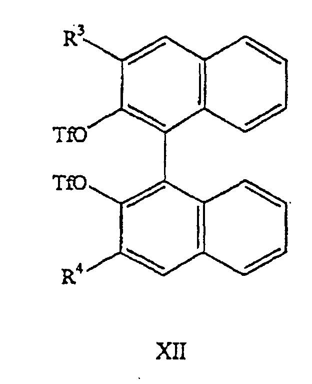 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₃ alkyl) carbamoyl group; and a N,N-di(C₁ to C₃ alkyl) carbamoyl group (wherein alkyl may be the same or different), and R³ and R⁴ may be the same or different; and Tf is a trifluoromethanesulfonyl group;
with a methylmagnesium halide represented by the formula (XIII):

MeMgX (XIII)

wherein X is a halogen atom;
in the presence of a nickel catalyst and in an appropriate solvent.

According to the present invention, the compound of the formula (XI) can be produced by stirring the compound of the formula (XII) with the compound of the formula (XIII) in the presence of a nickel catalyst in an ether solvent at an appropriate temperature. The compound of the formula (XIII) can be used preferably in 2 to 7 equivalents and more preferably 2.5 to 6.5 equivalents to the compound of the formula (XII). Examples of the compound (XIII) include MeMgCl. Examples of the nickel catalyst include NiCl₂(PPh₃)₂. Examples of the ether solvent include ether, isopropyl ether, butyl ether, THF and tert-butylmethyl ether. The temperature can be between -15°C and a boiling point of the solvent used, preferably between 0°C and 50°C. The stirring period can be preferably 2 to 50 hours and more preferably 5 to 40 hours. In the reaction, the solvent may be used in volume (mL) preferably 5 to 20 times, more preferably 7 to 15 times the weight (g) of the compound of the formula (XII). The nickel catalyst may be used preferably in 0.01 to 0.1 equivalents and more preferably 0.02 to 0.06 equivalents to the compound of the formula (XII).

When R³ and R⁴ are functional groups other than hydrogen atom, the compound of the formula (XII) can be produced by applying a method in which a reaction is catalyzed by palladium, such as a carbonylation reaction, Heck reaction, Stille reaction, Sonogashira reaction or Suzuki reaction to an optically active 3,3'-dibromo-2,2'-bistrifluoromethane-sulfonyloxy-1,1'-binaphthyl derivative represented by the formula (XIV): 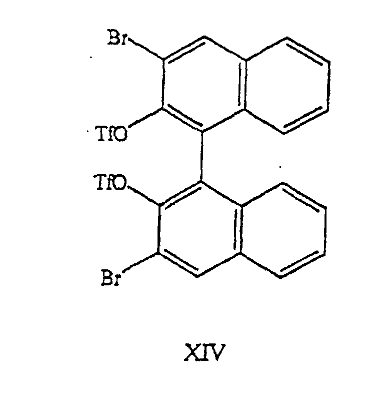 wherein Tf is a trifluoromethanesulfonyl group.

Particularly, the compound represented by the formula (XVII): 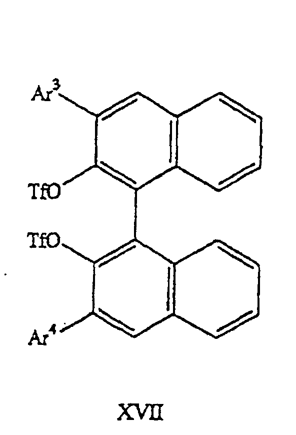 wherein Ar³ and Ar⁴ are groups selected from the group consisting of an alkenyl group having 2 to 6 carbon atoms which may be branched or form a cyclic group; an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar³ and Ar⁴ may be the same or different;
can be produced by reacting the compound of the formula (XIV) with a compound of the formula (XV):

Ar³B(OH)₂ (XV)

and a compound of the formula (XVI):

Ar⁴B(OH)₂ (XVI)

wherein, in the formulae (XV) and (XVI), Ar³ and Ar⁴ are groups selected from the group consisting of an alkenyl group having 2 to 6 carbon atoms which may be branched or form a cyclic group; an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms, or a halogen atom, and Ar³ and Ar⁴ may be the same or different; in this order or simultaneously, in an appropriate solvent and in the presence of a base and a palladium catalyst.

According to the present invention, the compound of the formula (XVII) can be produced by stirring the compound of the formula (XIV) with the compound of the formula (XV) and the compound of the formula (XVI) in an ether solvent or DMF in the presence of a palladium catalyst and a base at an appropriate temperature. The compounds of the formulae (XV) and (XVI) can be used each preferably in 1.2 to 3 equivalents, more preferably 1.2 to 2.0 equivalents and most preferably 1.25 to 1.75 equivalents to the compound of the formula (XIV). Examples of the ether solvent include ether, isopropyl ether, butyl ether, THF and tert-butylmethyl ether. Examples of the base include sodium bicarbonate, lithium hydroxide, sodium hydroxide, potassium hydroxide, thallium hydroxide, sodium methoxide, sodium ethoxide and potassium phosphate (hydrate). The palladium catalyst can be added preferably in 0.01 to 0.1 equivalents, more preferably 0.02 to 0.08 equivalents and most preferably 0.03 to 0.06 equivalents to the compound of the formula (XIV). The temperature can be between room temperature and a boiling point of the solvent used, preferably between room temperature and 100°C. The stirring period can be preferably 1 to 20 hours, more preferably 3 to 15 hours and most preferably 6 to 12 hours. In the reaction, the solvent may be used in volume (mL) preferably 5 to 20 times and more preferably 6 to 12 times the weight (g) of the compound of the formula (XIV). The base may be used preferably in 2 to 5 equivalents and more preferably 2.5 to 3.5 equivalents to the compound of the formula (XIV). Examples of the palladium catalyst include 0-equivalent palladium complexes such as Pd(PPh₃)₄ and a catalyst generated in situ from Pd(OAc)₂ and PPh₃. In the latter example, the ratio of Pd(OAc)₂ to PPh₃ can range from 1:4 to 1:5.

A compound of the formula (XIV) can be produced by reacting optically active 2,2'-dibromo-1,1'-bi-2-naphthol represented by the formula (XVIII): 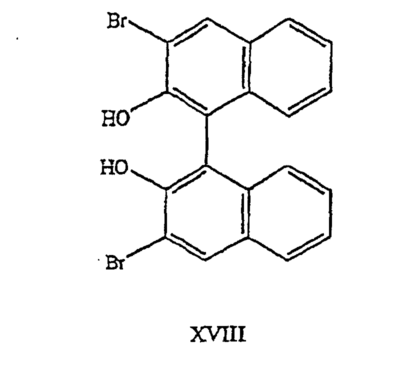 with a triflating agent in an inert solvent in the presence of a base at a temperature from -78°C to 0°C. Examples of the inert solvent include toluene, methylene chloride, THF and DMF. The base can be used preferably in 2 to 4 equivalents, more preferably 2.5 to 3.3 equivalents to the compound of the formula (XVIII). Examples of the triflating agent include trifluoromethanesulfonic anhydride and trifluoromethanesulfonyl chloride. The triflating agent can be used preferably in 2 to 2.5 equivalents, more preferably 2.2 to 2.5 equivalents to the compound of the formula (XVIII). In this reaction, the reaction solvent may be used in volume (mL) preferably 5 to 20 times and more preferably 6 to 15 times the weight (g) of the compound of the formula (XV). Examples of the base include a tertiary amine such as triethylamine, N,N-dimethylisopropylamine and N-methylmorpholine.

The optically active quarternary ammonium salts of the formulae (I) to (III) produced by the above methods having axial chirality and able to form spiro type structure are useful as a phase-transfer catalyst for stereoselective alkylation of the compound of the formula (XIX).

The compound of the formula (I) is pure regarding axial chirality, and used as a phase-transfer catalyst. The term "pure regarding axial chirality" means that the percentage of one of the isomer is larger than that of the other isomer(s) based on the axial chirality. The percentage of one isomer is 90 % or more, preferably 95 % or more, and even more preferably 98 % or more.

According to the present invention, the optically active compound (XXI) can be produced by stirring the compound (XIX) with any one of compounds (I) to (III) that can act as a phase-transfer catalyst in two-phase mixture comprising a hydrocarbon solvent and an alkaline aqueous solution at an appropriate temperature. The compound (XX) can be added preferably in 1 to 1.5 equivalents, more preferably 1.1 to 1.3 equivalents and most preferably 1.2 to 1.25 equivalents to the compound (XIX). The compound (I) to (III) can be added preferably in 0.005 to 0.03 equivalents and more preferably 0.0075 to 0.0125 equivalents to the compound (XIX). The temperature can be between -10°C and room temperature, preferably between -5°C and +5°C. The stirring period can be preferably 15 minutes to 3 hours, more preferably 0.5 to 2 hours and most preferably 0.5 to 1.5 hours. Using the above-mentioned methods, the optically active compound (XXI) can be produced in high yield and high optical purity.

For example, for the synthesis of an (S)-form of the compound (XXI), the compound of the formula (I) with an axial chirality of (S) can be used. On the other hand, for the synthesis of an (R) form of the compound (XXI), the compound of the formula (I) with an axial chirality of (R) can be used.

The term "high optical purity" used herein means optical purity of preferably 90 %e.e. or more and more preferably 95 % e.e. or more.

The hydrocarbon solvent used in the present invention includes any type of solvent which is immiscible with water, and includes hexane, toluene or the like. The solvent may be used in volume (mL) preferably 5 to 30 times and more preferably 8 to 25 times the weight (g) of the compound of the formula (XIX). For the alkaline aqueous solution, 10 to 60% aqueous solution of alkaline metal hydroxide such as lithium hydroxide, potassium hydroxide, sodium hydroxide, cesium hydroxide, rubidium hydroxide or the like may be used. The alkaline aqueous solution may be used at the volume (mL) to weight (g) ratio of preferably 4 to 20 and more preferably 8 to 15 based on the compound of the formula (XIX).

### EXAMPLES

Hereinafter, the present invention will be described more specifically by way of examples. However, the present invention is not limited by the Examples.

### Example 1

### Preparation of (S)-1,1'-bi-3-bromo-2-trifluoromethanesulfonyloxy-naphthyl (2)

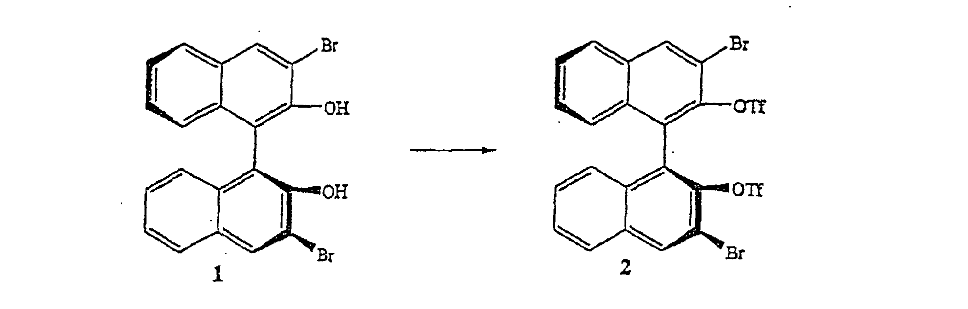

Under an argon atmosphere, triethylamine (6.54mL, 42mmol) was added to a solution of (S)-1,1'-bi-3-bromo-2-naphthol (1) (6.19g, 14mmol) in a dichloromethane (40mL) at room temperature, and cooled to -78°C. Then, trifluoromethanesulfonic anhydride (5.16mL, 31mmol) was added dropwise, and the mixture was stirred for 2 hours under the same cooling conditions. The reaction mixture was poured into a saturated NH₄Cl aqueous solution, and the mixture was extracted with dichloromethane. The dichloromethane extract was dried over Na₂SO₄, and concentrated under vacuum. The residue was chromatographed over silica gel. Elution with dichloromethane - hexane (1:5) gave (S)-1,1'-bi-3-bromo-2-trifluoromethanesulfonyloxynaphthyl (2) (9.90g, 14mmol) in a quantitative yield.

300MHz ¹H-NMR (CDCl₃): δ 8.44(2H, s, Ar-H), 7.92(2H, d, J=8.1Hz, Ar-H), 7.61(2H, ddd, J=1.2, 6.9, 8.1Hz, Ar-H), 7.41(2H, ddd, J=1.2, 6.9, 8.1Hz, Ar-H), 7.22(2H, d, J=8.1Hz, Ar-H) ppm.

### Example 2

### Preparation of (S)-1,1'-bi-3-(β-naphthyl)-2-trifluoromethanesulfonyloxynaphthyl (3)

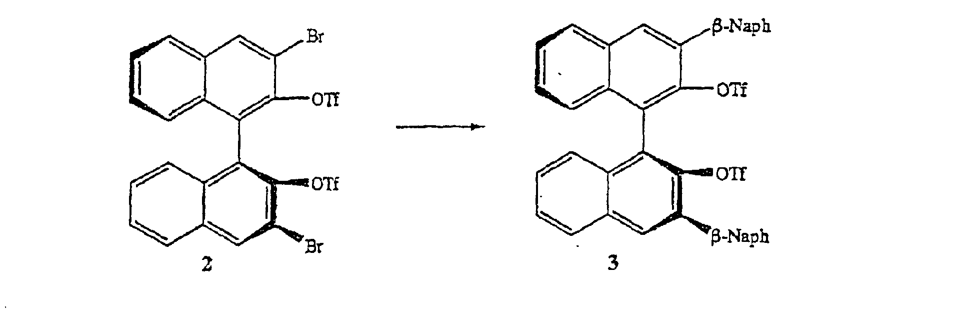

A mixture of (S)-1,1'-bi-3-bromo-2-trifluoromethanesulfonyloxynaphthyl (2) (3.54g, 5.0mmol), β-naphthylboronic acid (2.94g, 15mmol), palladium acetate [Pd(OAc)₂; 57.9mg, 5mol%], triphenylphosphine (0.294g, 22mol%), potassium phosphate·hydrate (4.29g, 15mmol) and THF (25mL) was stirred with heating at 65°C for 10 hours. Then, the reaction mixture was poured into a saturated NH₄Cl aqueous solution. The palladium catalyst was filtered off, and the filtrate was extracted with ether. The ether extract was dried over Na₂SO₄, and concentrated under vacuum. The residue was subjected to silica gel chromatography, and elution with ether - dichloromethane - hexane (1:2:60) gave (S)-1,1'-bi-3-( β -naphthyl)-2-trifluoromethane-sulfonyloxynaphthyl (3) (2.85g, 4.0mmol) in 80 % yield.

300MHz ¹H-NMR (CDCl₃): δ 8.23(2H, s, Ar-H), 8.15(2H, s, Ar-H), 7.94-8.05(8H, m, Ar-H), 7.77(2H, dd, J=1.8, 8.4Hz, Ar-H), 7.56-7.64(6H, m, Ar-H), 7.38-7.46(4H, m, Ar-H) ppm.

### Example 3

### Preparation of (S)-1,1'-bi-3-phenyl-2-trifluoromethanesulfonyloxynaphthyl (4)

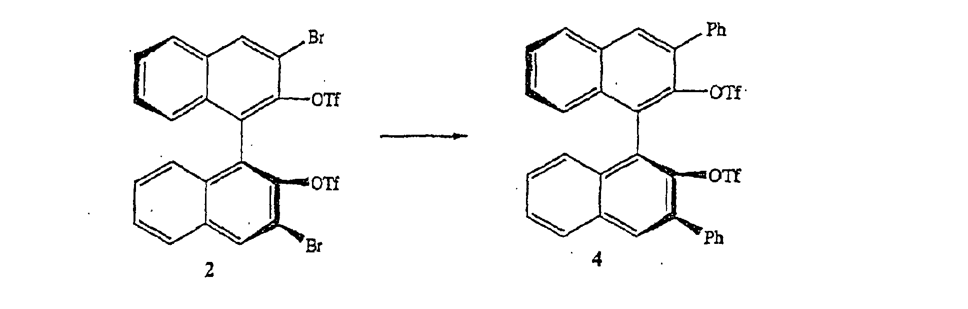

A mixture of (S)-1,1'-bi-3-bromo-2-trifluoromethanesulfonyloxynaphthyl (2) (3.54g, 5.0mmol), phenylboronic acid (1.83g, 15mmol), palladium acetate [Pd(OAc)₂; 57.9mg, 5mol%], triphenylphosphine (0.294g, 22mol%), potassium phosphate·hydrate (4.29g, 15mmol) and THF (25mL) was stirred with heating at 65°C for 10 hours. Then, the reaction mixture was poured into a saturated NH₄Cl aqueous solution. The palladium catalyst was filtered off, and the filtrate was extracted with ether. The ether extract was dried over Na₂SO₄, and then concentrated under vacuum. The residue was subjected to silica gel chromatography, and elution with ether - dichloromethane - hexane (1:2:60) gave (S)-1,1'-bi-3-phenyl-2-trifluoromethanesulfonyloxynaphthyl (4) (2.99g, 4.25 mmol) in 85 % yield.

300MHz ¹H-NMR (CDCl₃): δ 8.12(2H, s, Ar-H), 7.99(2H, d, J=8.1Hz, Ar-H), 7.34-7.66(16H, m, Ar-H) ppm.

### Example 4

### Preparation of (S)-1,1'-bi-2-methyl-3-(β-naphthyl)naphthyl (5)

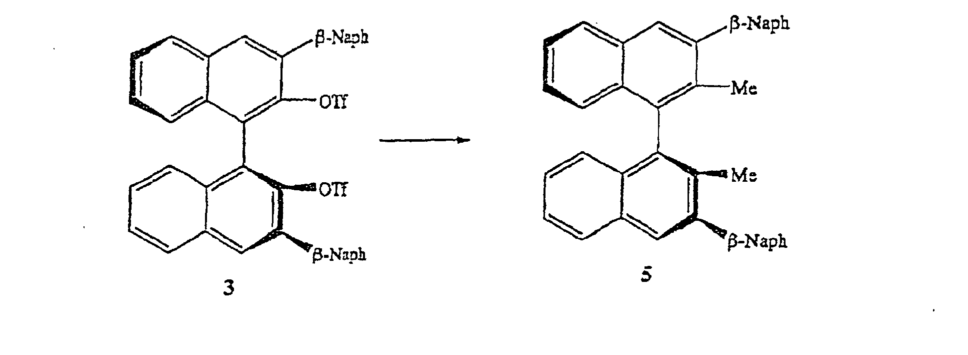

Under an argon atmosphere, a solution of MeMgI in ether (1.0M; 14mL, 14mmol) was added dropwise to a mixture of (S)-1,1'-bi-3-(β-naphthyl)-2-trifluoromethanesulfonyloxynaphthyl (3) (1.73g, 2.4mmol), bis(triphenylphosphine) nickel chloride [NiCl₂(PPh₃)₂; 78.5mg, 5mol%] and ether (4mL) at 0°C. The reaction mixture was stirred with heating at reflux for 30 hours, and poured into a saturated NH₄Cl aqueous solution. The mixture was extracted with ether, and the ether extract was filtrated to remove the nickel catalyst. The filtrate was washed with brine, dried over Na₂SO₄, and concentrated under vacuum. The residue was chromatographed over silica gel, and elution with ether - hexane (1:100) gave (S)-1,1'-bi-2-methyl-3-(β-naphthyl)naphthyl (5) (0.793g, 1.5mmol) in 62 % yield.

300MHz ¹H-NMR (CDCl₃): δ 7.91-7.98(10H, m, Ar-H), 7.63(2H, dd, J=1.8, 8.7Hz, Ar-H), 7.52-7.55(4H, m, Ar-H), 7.46(2H, ddd, J=1.2, 6.9, 8.1Hz, Ar-H), 7.30(2H, ddd, J=1.2, 6.9, 8.1Hz, Ar-H), 7.20(2H, d, J=8.7Hz, Ar-H), 2.03(6H, s, CH₃) ppm.

### Example 5

### Preparation of (S)-1,1'-bi-2-methyl-3-phenylnaphthyl (6)

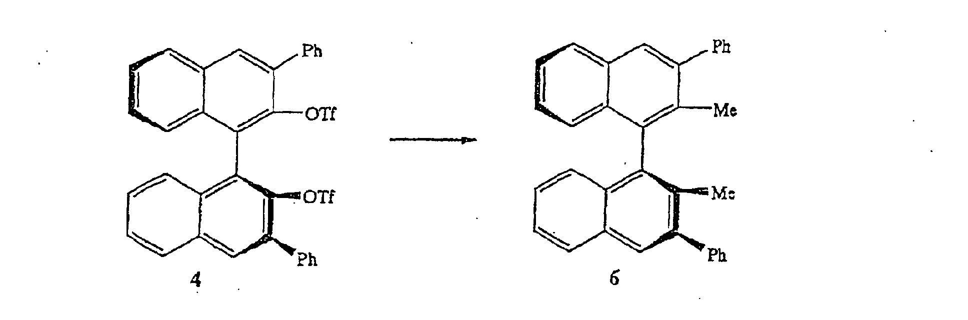

Under an argon atmosphere, a solution of MeMgI in ether (1.0M; 15mL, 15mmol) was added dropwise to a mixture of (S)-1,1'-bi-3-phenyl-2-trifluoromethanesulfonyloxy naphthyl (4) (1.72g, 2.5mmol), bis(triphenylphosphine) nickel chloride [NiCl₂(PPh₃)₂; 80.1mg, 5mol%] and ether (5mL) at 0°C. The reaction mixture was stirred with heating at reflux for 30 hours, and poured into a saturated NH₄Cl aqueous solution. The mixture was extracted with ether, and the ether extract was filtrated to remove the nickel catalyst. The filtrate was washed with brine, dried over Na₂SO₄, and concentrated under vacuum. The residue was chromatographed over silica gel, and elution with ether - hexane (1:100) gave (S)-1,1'-bi-2-methyl-3-phenylnaphthyl (6) (0.925g, 2.1mmol) in 87 % yield.

300MHz ¹H-NMR (CDCl₃): δ 7.85-7.91(4H, m, Ar-H), 7.36-7.51(12H, m, Ar-H), 7.25(2H, ddd, J=1.2, 8.4, 9.9Hz, Ar-H), 7.12(2H, d, J=8.4Hz, Ar-H), 1.95(6H, s, CH₃) ppm.

### Example 6

### Preparation of(S)-1,1'-bi-2-(bromomethyl)-3-(β-naphthyl)naphthyl (7)

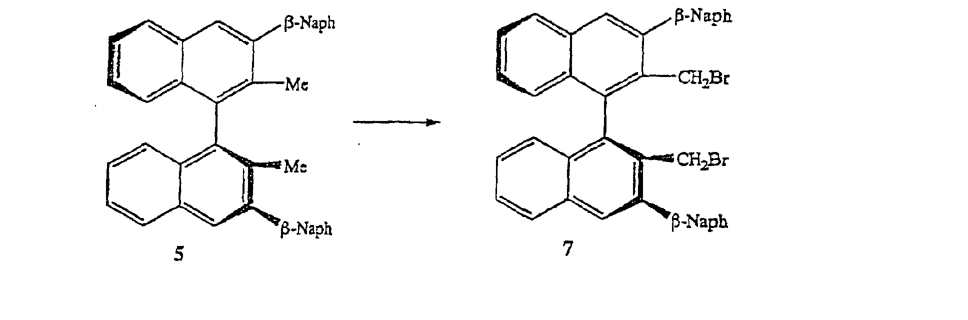

A mixture of (S)-1,1'-bi-2-methyl-3-(β-naphthyl)naphyl (5) (0.793g, 1.5mmol), N-bromosuccinimide (0.654g, 3.6mmol), benzoly peroxide (96.9mg, 0.3mmol) and cyclohexane (6mL) was stirred with heating at reflux for 3 hours, during which benzoyl peroxide (96.9mg, 0.3mmol) was added twice at one-hour interval. The reaction mixture was poured into a saturated sodium sulfite aqueous solution, and the mixture was extracted with ether. The ether extract was washed with brine, dried over Na₂SO₄, and then concentrated under vacuum. The residue was chromatographed over silica gel. Elution with ether-hexane (1:100) gave (S)-1,1'-bi-2-(bromomethyl)-3-phenylnaphthyl (7) (0.982g, 1.4mmol) in 95 % yield.

300MHz ¹H-NMR (CDCl₃): δ 8.12(2H, d, J=1.5Hz, Ar-H), 7.93-8.01(10H, m, Ar-H), 7.77(2H, dd, J=1.8, 8.4Hz, Ar-H), 7.52-7.57(6H, m, Ar-H), 7.34(2H, ddd, J=1.5, 6.9, 8.1Hz, Ar-H), 7.24(2H, d, J=9.0Hz, Ar-H), 4.36(4H, s, CH₂Br) ppm.

### Example 7

### Preparation of (S)-2,2'-bi-2-(bromomethyl)-3-phenylnaphthyl (8)

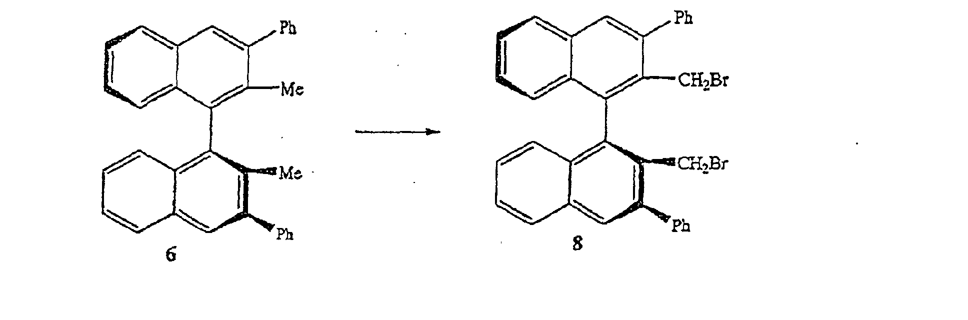

A mixture of (S)-1,1'-bi-2-methyl-3-phenylnaphthyl (6) (0.405g, 0.93mmol), N-bromosuccinimide (0.40g, 2.2mmol), benzoly peroxide (65.0mg, 0.2mmol) and cyclohexane (3mL) was stirred with heating at reflux for an hour. The reaction mixture was poured into a saturated sodium sulfite aqueous solution, and the mixture was extracted with ether. The ether extract was washed with brine, dried over Na₂SO₄, and concentrated under vacuum. The residue was subjected to silica gel chromatography, and elution with ether - hexane (1:100) gave (S)-1,1'-bi-2-(bromomethyl)-3-phenylnaphthyl (8) (0.55g, 0.93mmol) quantitatively.

300MHz ¹H-NMR (CDCl₃): δ 7.92(4H, t, J=8.1Hz, Ar-H), 7.61-7.65(4H, m, Ar-H), 7.45-7.55(8H, m, Ar-H), 7.30(2H, ddd, J=1.5, 6.9, 8.4Hz, Ar-H), 7.18(2H, d, J=7.2Hz, Ar-H), 4.29(4H, s, CH₂Br) ppm.

### Example 8

### Preparation of spiro bi[(S)-1,1'-binaphthyl-2,2'-dimethylammonium] bromide (11)

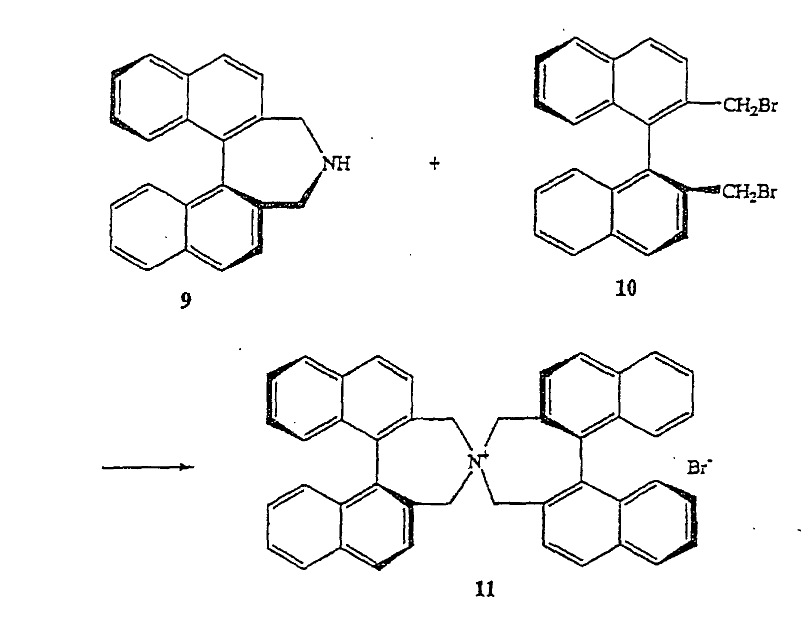

Potassium carbonate (0.417g, 3.0mmol) was added to a solution of (S)-3,5-dihydro-4H-[2,1-c:1',2'-e]azepine (9) (0.295g, 1.0mmol) in methanol (3mL), and the mixture was stirred at room temperature for 30 minutes. Then, (S)-1,1'-bi-2-(bromomethyl)naphthyl (10) (0.44g, 1.0mmol) was added. The reaction mixture was stirred with heating at reflux for 8 hours, and poured into water. The mixture was extracted with dichloromethane. The dichloromethane extract was dried over Na₂SO₄, and concentrated under vacuum. The residue was subjected to silica gel chromatography, and elution with methanol - dichloromethane (1:30) gave compound (11) (0.465g, 1.71mmol) in 71 % yield.

300MHz ¹H-NMR (CDCl₃): δ 8.38(4H, d, J=8.1Hz, Ar-H), 8.17(4H, d, J=6.6Hz, Ar-H), 8.11(4H, d, J=6.6Hz, Ar-H), 7.64(4H, ddd, J=1.4, 6.6, 8.1Hz, Ar-H), 7.26-7.44(8H, m, Ar-H), 4.52(4H, d, J=13.2Hz, ArCH₂), 3.92(4H, d, J=13.2Hz, ArCH₂) ppm; IR (KBr): ν 3647, 3400, 3053, 2361, 1624, 1595, 1508, 1458, 1346, 1030, 862, 822, 756cm⁻¹; MS: 574(M⁺)(100%).

### Example 9

Preparation of [(S)-3,3'-diphenyl-1,1'-binaphthyl-2,2'-dimethyl ammonium] spiro [(S)-1,1'-binaphthyl-2,2'-dimethylamine]bromide (12)

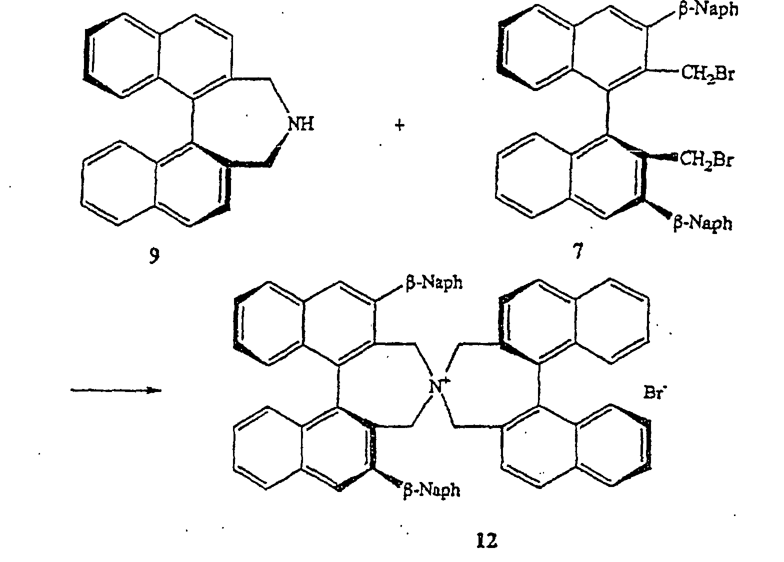

Potassium carbonate (0.208g, 1.5mmol) was added to a solution of (S)-3,5-dihydro-4H-[2,1-c:1',2'-e]azepine (9) (0.148g, 0.5mmol) in methanol (3mL), and the mixture was stirred at room temperature for 30 minutes. Then, (S)-1,1'-bi-2-(bromomethyl)-3-(β-naphthyl)naphthyl (7) (0.346g, 0.5mmol) was added. The reaction mixture was stirred with heating at reflux for 8 hours, and poured into water. The mixture was extracted with dichloromethane. The dichloromethane extract was dried over Na₂SO₄, and concentrated under vacuum. The residue was subjected to silica gel chromatography, and elution with methanol-dichloromethane (1:30) gave compound (12) (0.162g, 0.17mmol) in 36 % yield.

300MHz ¹H-NMR (CDCl₃): δ 8.49(2H, s, Ar-H), 8.16(2H, d, J=8.4Hz, Ar-H), 8.14(2H, br, Ar-H), 7.79(2H, br, β-Np), 7.67(2H, t, J=6.9Hz, Ar-H), 7.31-7.39(4H, m, Ar-H), 7.20(2H, d, J=7.5Hz, Ar-H), 7.08(2H, t, J=6.8Hz, Ar-H), 6.94(2H, d, J=9.0Hz, Ar-H), 7.0-8.6(12H, br, β-Np), 5.05(2H, br, ArCH₂), 4.50(2H, d, J=13.8Hz, ArCH₂), 4.22(2H, d, J=12.9Hz, ArCH₂), 3.66(2H, d, J=12.9Hz, ArCH₂) ppm; IR (KBr): ν 3852, 3649, 3367, 3051, 1653, 1558, 1506, 1456, 1361, 853, 833, 749cm⁻¹.

### Example 10

### Preparation of [(S)-3,3'-di(β-naphthyl)-1,1'-binaphthyl-2,2'-dimetylammonium]spiro[(S)-1,1'-binaphthyl-2,2'-dimethylamine]bromide (13)

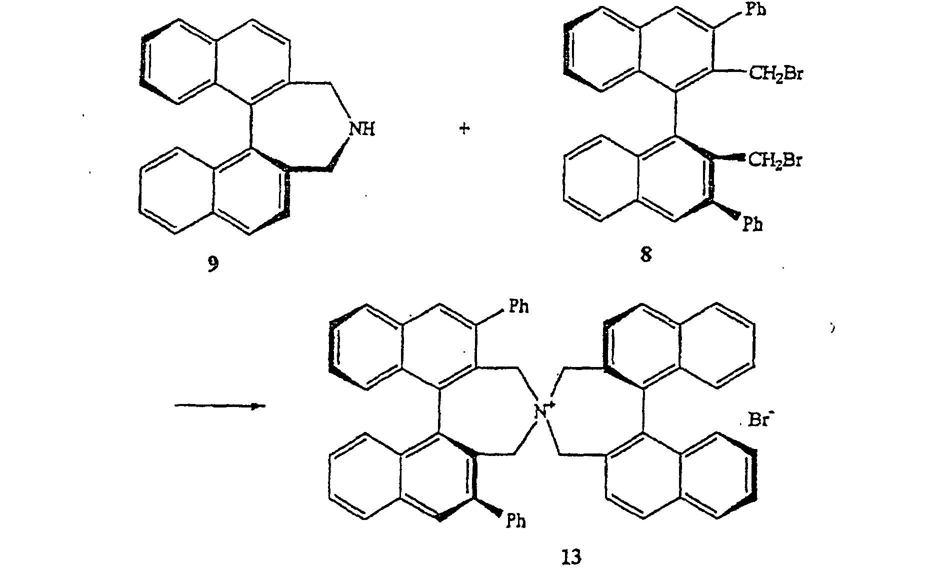

Potassium carbonate (83.0mg, 0.6mmol) was added to a solution of (S)-3,5-dihydro-4H-[2,1-c:1',2'-e]azepine (9) (89mg, 0.3mmol) in methanol (3mL), and the mixture was stirred at room temperature for 30 minutes. Then, (S)-1,1'-bi-2-(bromomethyl)-3-phenylnaphthyl (8) (0.178g, 0.3mmol) was added. The reaction mixture was stirred with heating at reflux for 8 hours, and poured into water. The mixture was extracted with dichloromethane. The dichloromethane extract was dried over Na₂SO₄, and then concentrated under vacuum. The residue was subjected to silica gel chromatography, and elution with methanol - dichloromethane (1:30) gave compound (13) (0.196g, 0.24mmol) in 81 % yield.

300MHz ¹H-NMR (CDCl₃): δ 8.34(2H, s), 8.11(2H, d, J=8.1Hz, Ar-H), 7.84(2H, d, J=8.1Hz, Ar-H), 7.74(2H, br, Ph), 7.63(2H, ddd, J=1.1, 7.2, 8.0Hz, Ar-H), 7.49(2H, ddd, J=1.1, 7.2, 8.0Hz, Ar-H), 7.31-7.36(4H, m, Ar-H), 7.09-7.22(6H, m, Ar-H), 7.2-8.2(8H, br, Ph), 6.32(2H, d, J=8.4Hz, Ar-H), 5.01(2H, d, J=13.7Hz, ArCH₂), 4.40(2H, d, J=13.2Hz, ArCH₂), 4.24(2H, d, J=13.7Hz, ArCH₂), 3.71(2H, d, J=13.2Hz, ArCH₂) ppm; IR (KBr): ν 3649, 3367, 3053, 1653, 1558, 1491, 1456, 847, 812, 752, 708cm⁻¹.

### Example 11

### Preparation of (S)-N,N-di(β-naphthyl)-3,5-dihydro-4H-[2,1-c:1',2'-e] azepinium bromide (14a)

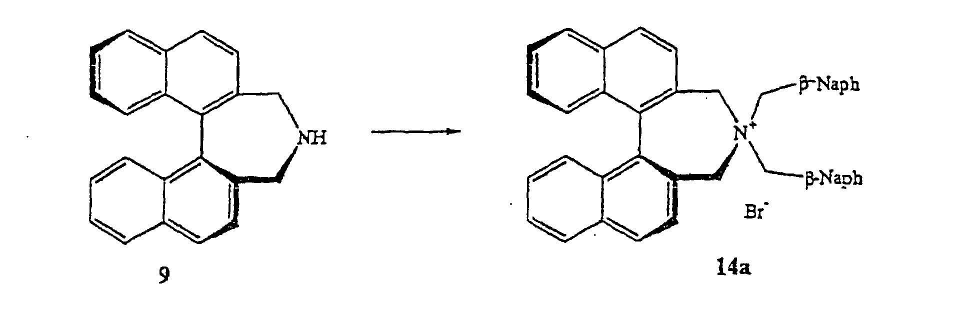

Potassium carbonate (0.139g, 1.0mmol) was added to a solution of (S)-3,5-dihydro-4H-[2,1-c:1',2'-e]azepine (0.148g, 0.5mmol) in methanol (3mL), and the mixture was stirred at room temperature for 30 minutes. Then, 2-(bromomethyl)naphthalene (0.276g, 1.2mmol) was added. The reaction mixture was stirred with heating at reflux for 3 hours, and poured into water. The mixture was extracted with dichloromethane. The dichloromethane extract was dried over Na₂SO₄ and concentrated under vacuum. The residue was subjected to silica gel chromatography, and elution with methanol - dichloromethane (1:30) gave the compound (14a) (0.220g, 0.34mmol) in 68 % yield.

300MHz ¹H-NMR (CDCl₃): δ 8.05(2H, s, Ar-H), 7.81-7.86(6H, m, Ar-H), 7.75(2H, d, J=8.7Hz, Ar-H), 7.49-7.66(10H, m, Ar-H), 7.23-7.31(6H, m, Ar-H), 6.07(2H, d, J=13.2Hz, ArCH₂), 5.38(2H, d, J=13.2Hz, ArCH₂), 4.85(2H, d, J=12.9Hz, ArCH₂), 4.42(2H, d, J=12.9Hz, ArCH₂) ppm.

### Example 12

### Preparation of (S)-N,N-di(α-naphthyl)-3,5-dihydro-4H-[2,1-c:1',2'-e] azepinium bromide (14b)

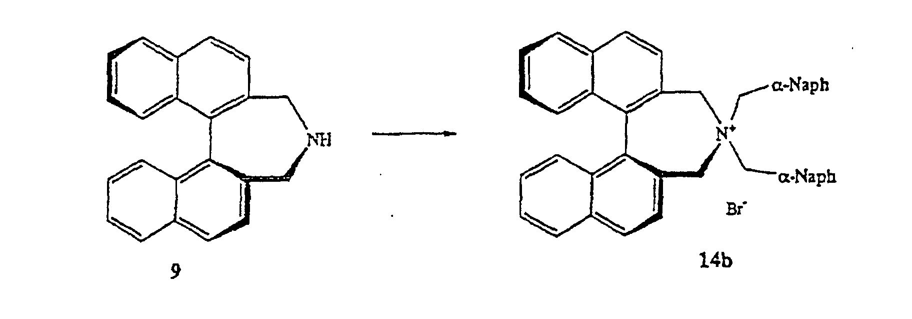

According to the method described in Example 11, compound (14b) was obtained from (S)-3,5-dihydro-4H-[2,1-c:1',2'-e]azepine and 1-(bromo-methyl)naphthalene in 24 % yield.

300MHz ¹H-NMR (CDCl₃): δ 7.96(4H, dd, J=8.1, 18.0Hz, Ar-H), 7.89(4H, d, J=8.4Hz, Ar-H), 7.74-7.62(6H, m, Ar-H), 7.56-7.46(4H, m, Ar-H), 7.39-7.18(6H, m, Ar-H), 6.89(2H, d, J=8.1Hz, Ar-H), 6.39(2H, d, J=13.8Hz, ArCH₂), 5.51(2H, d, J=13.8Hz, ArCH₂), 5.30(2H, d, J=13.2Hz, ArCH₂), 4.50(2H, d, J=13.2Hz, ArCH₂) ppm.

### Example 13

### Preparation of (S)-N,N-dibenzyl-3,5-dihydro-4H-[2,1-c;1',2'-e]azepinium bromide (15)

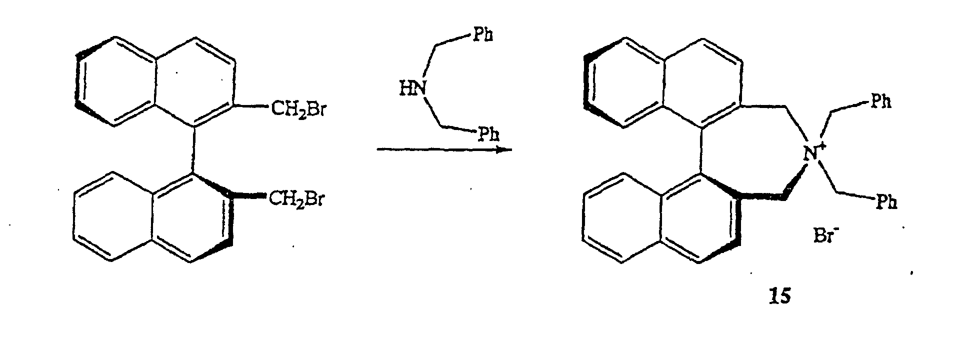

Potassium carbonate (55mg, 0.4mmol) was added to a solution of dibenzylamine (39mL, 0.2mmol) in methanol (3mL), and the mixture was stirred at room temperature for 30 minutes. Then, (S)-1,1'-bi-2-(bromomethyl)naphthyl (88mg, 0.2mmol) was added. The reaction mixture was stirred with heating at reflux for 4 hours, and poured into water. The mixture was extracted with dichloromethane. The dichloromethane extract was dried over Na₂SO₄, and concentrated under vacuum. The residue was chromatographed over silica gel, and elution with methanol - dichloromethane (1:30) gave compound (15) (55mg, 0.1mmol) in 50 % yield.

300MHz ¹H-NMR (CDCl₃): δ 7.95(2H, d, J=8.1Hz, Ar-H), 7.87(2H, d, J=8.7Hz, Ar-H), 7.54-7.65(8H, m, Ar-H), 7.27-7.43(10H, m, Ar-H, 5.76(2H, d, J=13.2Hz, ArCH₂), 5.20(2H, d, J=12.9Hz, ArCH₂), 4.69(2H, d, J=13.2Hz, ArCH₂), 4.30(2H, d, J=12.9Hz, ArCH₂) ppm.

### Example 14

### Preparation of (S)-phenylalanine tert-butylester benzophenone Schiff base (17)

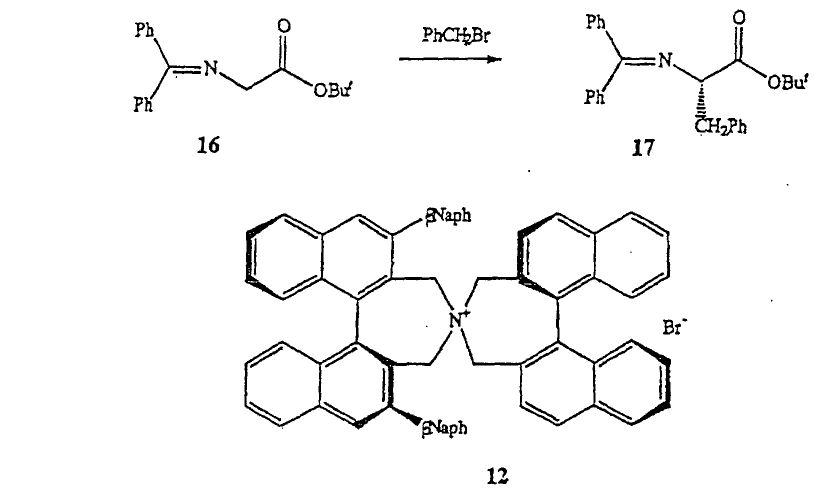

Benzyl bromide (72.1 µL, 0.6mmol) was added dropwise to a mixture of glycine tert-butylester benzophenone Schiff base (16) (148mg, 0.5mmol), asymmetric phase-transfer catalyst (12) (45mg, 0.005mmol), toluene (3.25mL) and 50% potassium hydroxide aqueous solution (1.05mL) at 0°C. The mixture was stirred at 0°C for 30 minutes and poured into water. The mixture was extracted with ether. Then, the ether extract was washed with brine, dried over Na₂SO₄, and concentrated under vacuum. The oily residue was subjected to silica gel chromatography, and elution with ether - hexane (1:10) gave (S)-phenylalanine tert-butylester benzophenone Schiff base (17) (183mg, 0.475mmol) in 95 % yield, which showed optical puritty of 96 %e.e. as demonstrated by HPLC analysis; DAICEl CHIRAL OD; hexane:2-propanol (100:1), 0.5 mL/min.; (R) form:14.8 min., (S) form:28.2 min.

### Examples 15 - 23

Other examples of the steroselective alkylation of glycine tert-butylester benzophenone Schiff base (16) using asymmetric phase-transfer catalyst (12) in the same manner as in Example 14, are listed in Table 1.

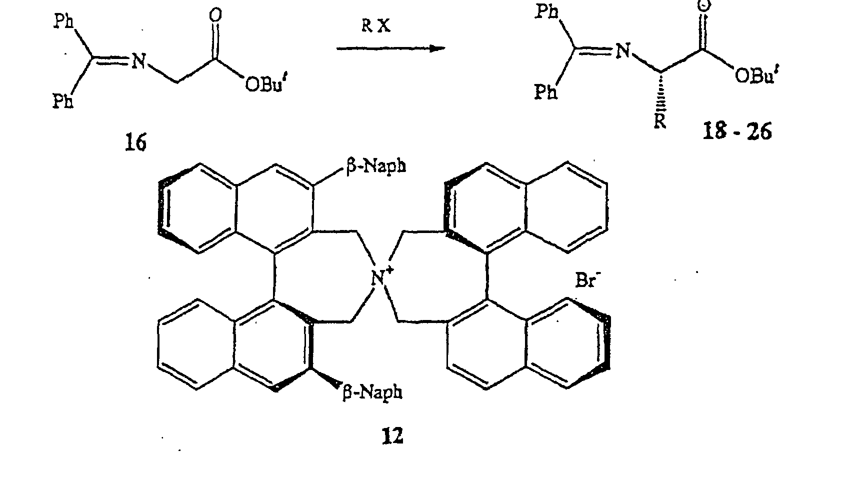

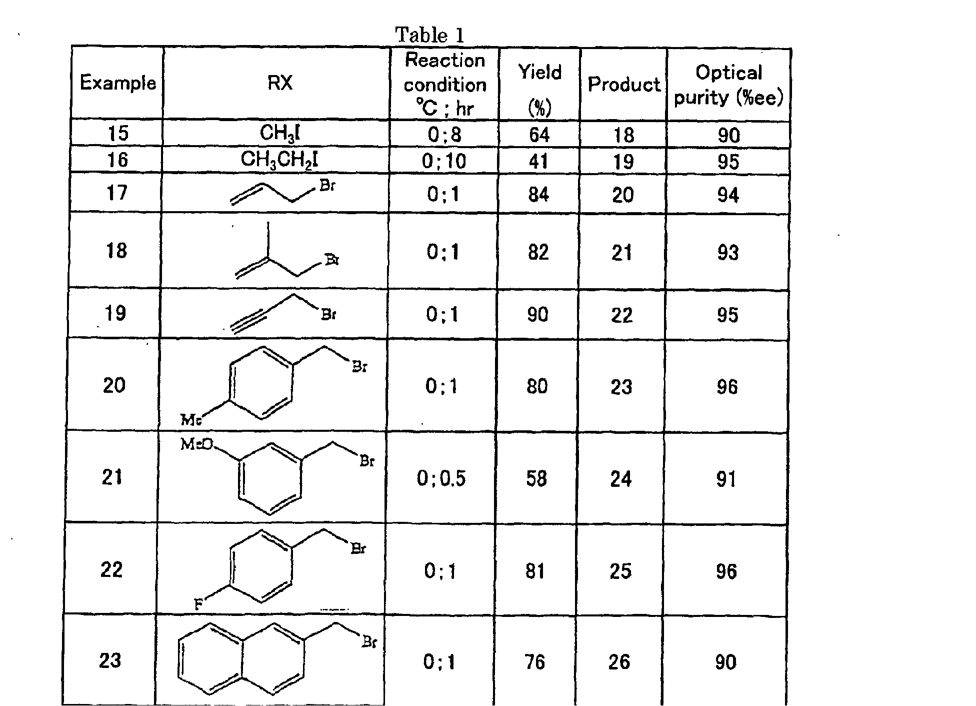

As shown in Table 1, when the present optically active quarternary ammonium salts with axial chirality are used as asymmetric phase-transfer catalysts, it is found that glycine tert-butylester benzophenone Schiff base can be stereoselectively alkylated in high optical purity.

### Examples 24-26

Other examples of the stereoselective alkylation of glycine tert-butylester benzophenone Schiff base (16) with benzyl bromide using asymmetric phase-transfer catalyst (14a), (14b), or (15) in the same manner as in Example 14, are listed in Table 2.

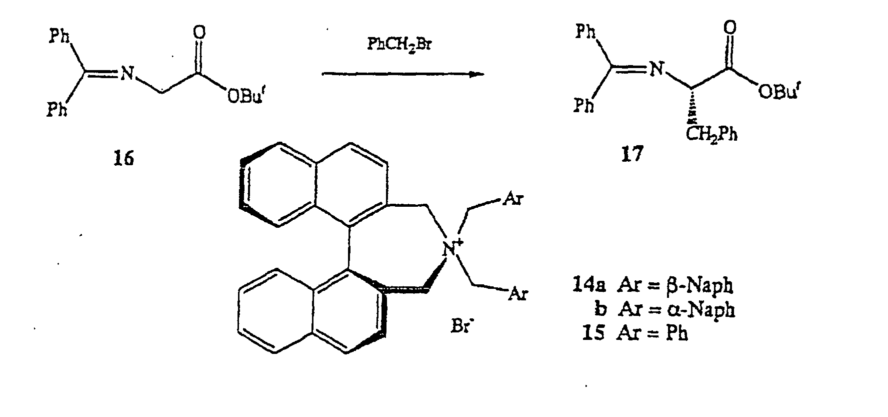

**Table 2**

| Example | Phase-tranfer catalyst | Reaction condition °C ; hr | Yield (%) | Product | Optical purity (%ee) |
|---|---|---|---|---|---|
| 24 | 14a | 0:6 | 44 | 17 | 17 |
| 25 | 14b | 0:8 | 46 | 17 | 28 |
| 26 | 15 | 0:6 | 34 | 17 | 21 |

### Example 27

### Preparation of (S)-1,1'-bi-3-hydroxy-2-methoxymethoxynaphthyl (28)

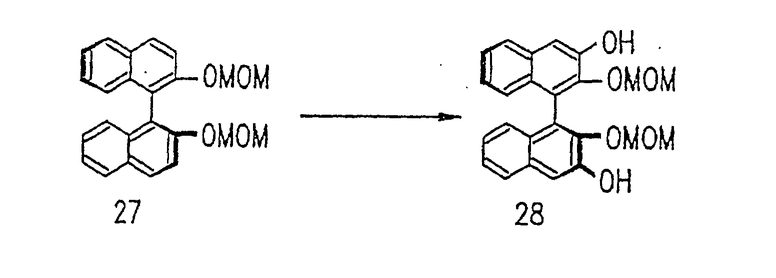

Under an argon atmosphere, a solution of n-butyllithium in hexane (1.60M, 30.0mL, 48mmol) was added dropwise to a solution of compound (27) (7.50g, 20mmol; prepared by the method of Katsuki et al., Chem. Lett. 1995, 1113) in ether (120mL) at room temperature. After stirrring for 4 hours, the reaction mixture was cooled to -78°C, and THE (150mL) was added. After trimethoxyborane (6.73mL, 60mmol) was added dropwise, the mixture was allowed to warm to room temperature, and then stirred for 10 hours. The reaction mixture was concentrated under vacuum with a rotary evaporator, and benzene (100mL) was added. The mixture was cooled to 0°C, and then hydrogen peroxide solution (30%, 10mL) was added dropwise. The reaction mixture was stirred with heating at reflux for 2 hours, and poured into a saturated Na₂SO₃ aqueous solution. The mixture was extracted with ether. The ether extract was washed with brine, dried over Na₂SO₄, and concentrated under vacuum. The residue was subjected to silica gel chromatography, and elution with ethyl acetate - hexane (1:2) gave compound (28) (6.05g, 15mmol) in 75 % yield.

300MHz ¹H-NMR (CDCl₃): δ 7.78(2H, d, J=8.4Hz, Ar-H), 7.51(2H, s, Ar-H), 7.45(2H, s, ArOH), 7.34(2H, ddd, J=1.2, 6.9, 7.8Hz, Ar-H), 7.12(2H, ddd, J=1.2, 6.9, 7.8Hz, Ar-H), 7.04(2H, d, J=8.4Hz, Ar-H), 4.72(2H, d, J=6.3Hz, ArOCH₂), 4.64(2H, d, J=6.3Hz, ArOCH₂), 3.40(6H, s, OCH₃) ppm.

### Example 28

### Preparation of (S)-1,1'-bi-3-methoxy-2-methoxymethoxynaphthyl (29)

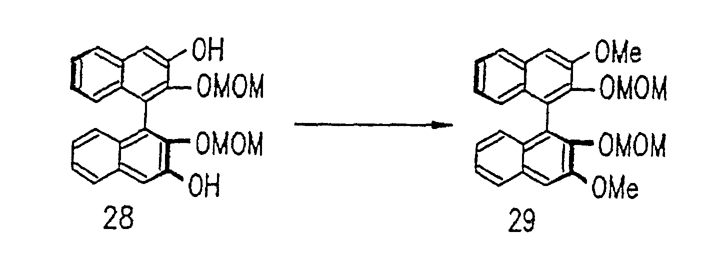

A mixture of compound (28) (6.05g, 15mmol), potassium carbonate (6.25g, 45mmol), methyl iodide (4.86mL, 75mmol) and acetone (200mL) was stirred with heating at reflux for 6 hours. Then, the reaction mixture was poured into water, and the mixture was extracted with ether. The ether extract was washed with brine, dried over Na₂SO₄, and then concentrated under vacuum. The residue was subjected to silica gel chromatography, and elution with ethyl acetate - hexane (1:3) gave compound (29) (5.60g, 13mmol) in 86 % yield.

300MHz ¹H-NMR (CDCl₃): δ 7.76(2H, d, J=8.1Hz, Ar-H), 7.36(2H, ddd, J=1.2, 6.0, 8.1Hz, Ar-H), 7.30(2H, s, Ar-H), 7.10-7.18(4H, m, Ar-H), 4.97(2H, d, J=5.7Hz, ArOCH₂), 4.83(2H, d, J=5.7Hz, ArOCH₂), 4.03(6H, s, ArOCH₃), 2.57(6H, s, OCH₃) ppm.

### Example 29

### Preparation of (S)-1,1'-bi-2-hydroxy-3-methoxynaphthyl (30)

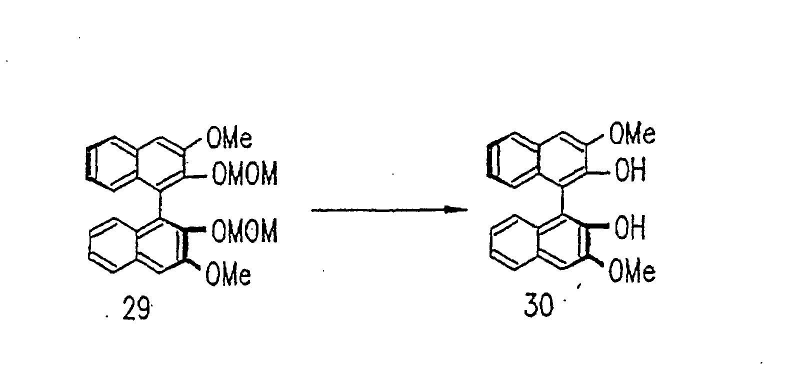

A mixture of compound (29) (5.60g, 13mmol), 1,4-dioxane (40mL) and concentrated hydrochloric acid (1mL) was stirred with heating at 50°C for 4 hours. Then, the reaction mixture was poured into water, and the mixture was extracted with ether. The ether extract was washed with water and brine, dried over Na₂SO₄, and concentrated under vacuum. The residue was chromatographed over silica gel. Elution with ethyl acetate - hexane (1:1) gave compound (30) (4.50g, 13mmol) in a quantitative yield.

300MHz ¹H-NMR (CDCl₃): δ 7.78(2H, d, J=8.1Hz, Ar-H), 7.32(2H, ddd, J=2.4, 5.7, 8.4Hz, Ar-H), 7.30(2H, s, Ar-H), 7.12-7.19(4H, m, Ar-H), 5.89(2H, s, ArOH), 4.10(6H, s, ArOCH₃), ppm.

### Example 30

### Preparation of (S)-1,1'-bi-3-methoxy-2-trifluoromethanesulfonyloxynaphthyl (31)

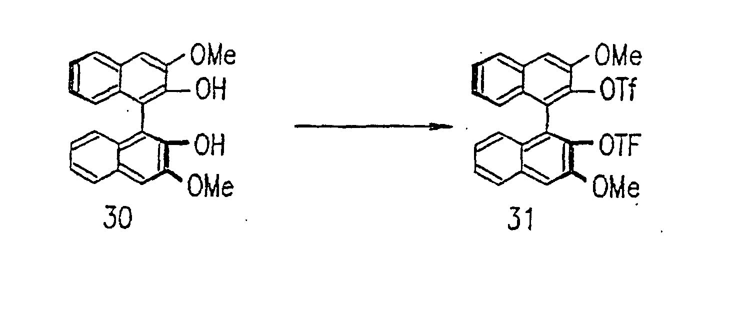

Under an argon atmosphere, triethylamine (5.52mL, 39mmol) was added to a solution of compound (30) (4.50g, 13mmol) in dichloromethane (50mL) at room temperature. Then, the solution was cooled to -78°C. After trifluoromethanesulfonic anhydrade (5.17mL, 31mmol) was added dropwise, the reaction mixture was allowed to warm to room temperature and stirred for 2 hours. The reaction mixture was poured into a saturated NH₄Cl aqueous solution, and the mixture was extracted with dichloromethane. The dichloromethane extract was dried over Na₂SO₄, and concentrated under vacuum. The residue was subjected to silica gel chromatography, and elution with dichloromethane - hexane (1:3) gave compound (31) (7.72g, 13mmol) in a quantitative yield.

300MHz ¹H-NMR (CDCl₃): δ 7.87(2H, d, J=8.4Hz, Ar-H), 7.52(2H, ddd, J=1.2, 6.9, 8.4Hz, Ar-H), 7.49(2H, s, Ar-H), 7.24(2H, ddd, J=1.2, 6.9, 7.8Hz, Ar-H), 7.14(2H, d, J=7.8Hz, Ar-H), 4.12(6H, s, ArOCH₃) ppm.

### Example 31

### Preparation of (S)-1,1'-bi-3-methoxy-2-methylnaphthyl (32)

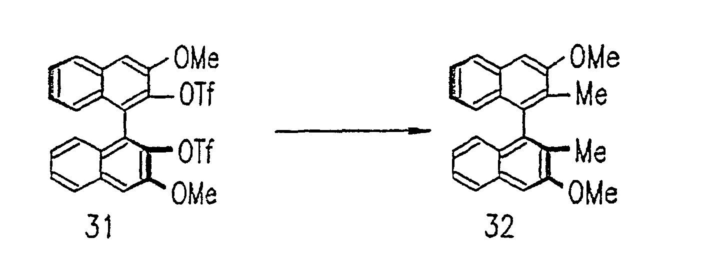

Under an argon atmosphere, a solution of MeMgI in ether (1.0M, 75mL, 75mmol) was added dropwise to a mixuture of compound (31) (7.72g, 13mmol), [1,3-bis(diphenylphosphino)propane] nickel chloride [NiCl₂(dppp), 342mg, 5mol%], and ether (20mL) at 0°C. The reaction mixture was stirred at room temperature for 30 hours, and then poured into saturated NH₄Cl aqueous solution. The nickel catalyst was filtered off, and the filtrate was extracted with ether. The ether extract was washed with brine, dried over Na₂SO₄ and concentrated under vacuum. The residue was chromatographed over silica gel. Elution with ether - hexane (1:10) gave compound (32) (3.40g, 9.9mmol) in 76 % yield.

300MHz ¹H-NMR (CDCl₃): δ 7.80(2H, d, J=8.1Hz, Ar-H), 7.36(2H, ddd, J=1.2, 6.9, 8.1Hz, Ar-H), 7.26(2H, s, Ar-H), 7.06(2H, ddd, J=1.2, 6.9, 8.1Hz, Ar-H), 6.96(2H, d, J=8.1Hz, Ar-H), 4.03(6H, s, ArOCH₃), 1.92(6H, s, ArCH₃) ppm.

### Example 32

### Preparation of (S)-1,1'-bi-3-hydroxy-2-methylnaphthyl (33)

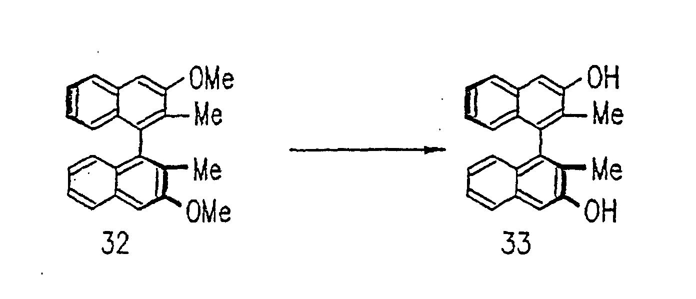

Under an argon atmosphere, boron tribromide (2.27mL, 24mmol) was added dropwise to a solution of compound (32) (3.40g, 9.9mmol) in dichloromethane (40mL) at 0°C. The reaction mixture was allowed to warm to room temperature and stirred for 2 hours. The mixture was then cooled to 0°C again, and water was added dropwise. The mixture was extracted with dichloromethane. The dichloromethane extract was washed with brine, dried over Na₂SO₄, and concentrated under vacuum. The residue was subjected to silica gel chromatography, and elution with ethyl acetate - hexane (1:1) gave compound (33) (3.13g, 9.9mmol) in a quantitative yield.

300MHz ¹H-NMR (CDCl₃): δ 7.74(2H, d, J=8.4Hz, Ar-H), 7.36(2H, ddd, J=1.5, 6.9, 8.1Hz, Ar-H), 7.27(2H, s, Ar-H), 7.07(2H, ddd, J=1.5, 6.9, 8.4Hz, Ar-H), 6.96(2H, d, J=8.1Hz, Ar-H), 5.14(6H, s, ArOH), 1.97(6H, s, ArCH₃) ppm.

### Example 33

### Preparation of (S)-1,1'-bi-2-methyl-3-trifluoromethanesulfonyloxynaphthyl (34)

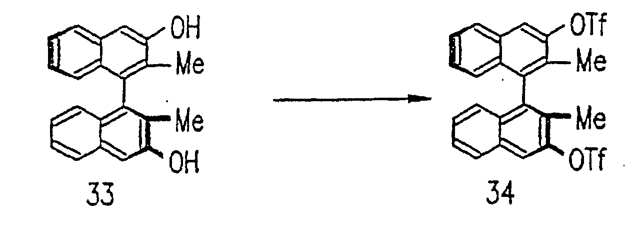

Under an argon atomosphere, triethylamine (4.20mL, 30mmol) was added to a solution of compound (33) (3.13g, 9.9mmol) in dichloromethane (30mL) at room temperature. Then, the solution was cooled to -78°C. After trifluoromethanesulfonic anhydrade (4.04mL, 24mmol) was added dropwise, the reaction mixture was allowed to warm to room temperature and stirred for 2 hours. The reaction mixture was poured into a saturated NH₄Cl aqueous solution, and the mixture was extracted with dichloromethane. The dichloromethane extract was dried over Na₂SO₄, and concentrated under vacuum. The residue was subjected to silica gel chromatography, and elution with dichloromethane - hexane (1:5) gave compound (34) (5.44g, 9.4mmol) in 95% yield.

300MHz ¹H-NMR (CDCl₃): δ 7.96(2H, d, J=8.4Hz, Ar-H), 7.94(2H, s, Ar-H), 7.54(2H, ddd, J=1.2, 6.9, 8.4Hz, Ar-H), 7.34(2H, ddd, J=1.2, 6.9, 8.4Hz, Ar-H), 6.99(2H, d, J=8.4Hz, Ar-H), 2.04(6H, s, ArCH₃) ppm.

### Example 34

### Preparation of (S)-1,1'-bi-2-methyl-3-(3",4",5"-trifluorophenyl)naphthyl (35)

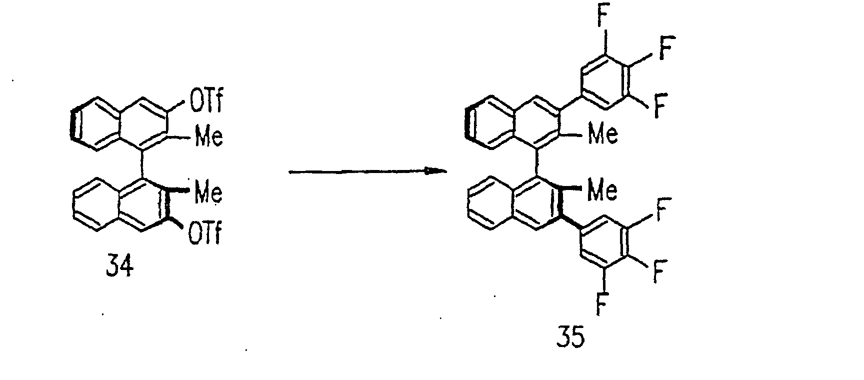

Under an argon atmosphere, a mixture of compound (34) (289mg, 0.50mmol), 3,4,5-trifluorophenyl boronic acid (211mg, 1.2mmol), tetrakis(triphenylphosphine) palladium (28.9mg, 5mol%), potassium phosphate·hydrate (429mg, 1.5mmol) and dioxane (5mL) was stirred with heating at 80°C for 10 hours. Then, the reaction mixture was poured into brine. The palladium catalyst was filtered off and the filtrate was extracted with ether. The ether extract was dried over Na₂SO₄ and concentrated under vacuum. The residue was subjected to silica gel chromatography, and elution with dichloromethane - hexane (1:20) gave compound (35) (253mg, 0.47mmol) in 94% yield. 300MHz ¹H-NMR (CDCl₃): δ 7.91(2H, d, J=8.1Hz, Ar-H), 7.82(2H, s, Ar-H), 7.47(2H, ddd, J=1.2, 6.9, 8.1Hz, Ar-H), 7.29(2H, ddd, J=1.2, 6.9, 8.4Hz, Ar-H), 7.05-7.14(6H, m, Ar-H), 1.91(6H, s, ArCH₃) ppm.

### Example 35

### Preparation of (S)-1,1'-bis-2-bromomethyl-3-(3",4",5"-trifluorophenyl) naphthyl (36)

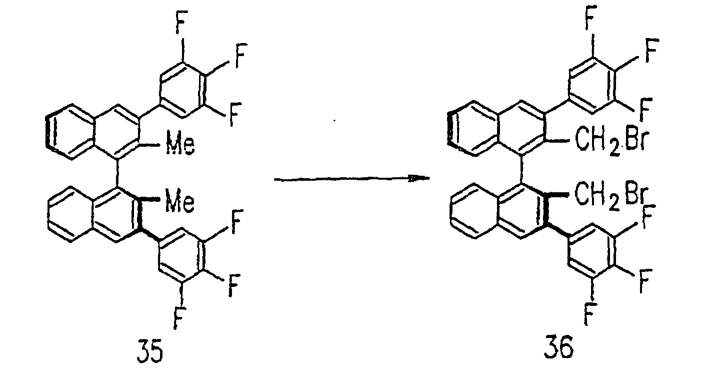

A mixture of compound (35) (253mg, 0.47mmol), 2,2'-azobis (isobutylonitrile) (7.9mg, 10mol%), N-bromosuccinimide (188mg, 1.0mmol) and benzene (4mL) was stirred with heating at reflux for 2 hours. The reaction mixture was poured into water, and the mixture was extracted with ether. The ether extract was dried over Na₂SO₄ and concentrated under vacuum. The residue was chromatographed over silica gel. Elution with ether - hexane (1:20) gave compound (36) (309mg, 0.44mmol) in 94% yield.

300MHz ¹H-NMR (CDCl₃): δ 7.94(2H, d, J=8.1Hz, Ar-H), 7.89(2H, s, Ar-H), 7.57(2H, ddd, J=1.2, 6.9, 8.1Hz, Ar-H), 7.34(2H, ddd, J=1.2, 6.9, 8.1Hz, Ar-H), 7.25-7.30(4H, m, Ar-H), 7.13(2H, d, J=8.1Hz, Ar-H), 4.19(4H, s, CH₂Br) ppm.

### Example 36

### Preparation of [(S)-3,3'-di(3",4",5"-trifluorophenyl)-1,1'-binaphthyl-2,2'-dimethylammonium] spiro [(S)-1,1'-binaphthyl-2,2'-dimethylamine] bromide (38)

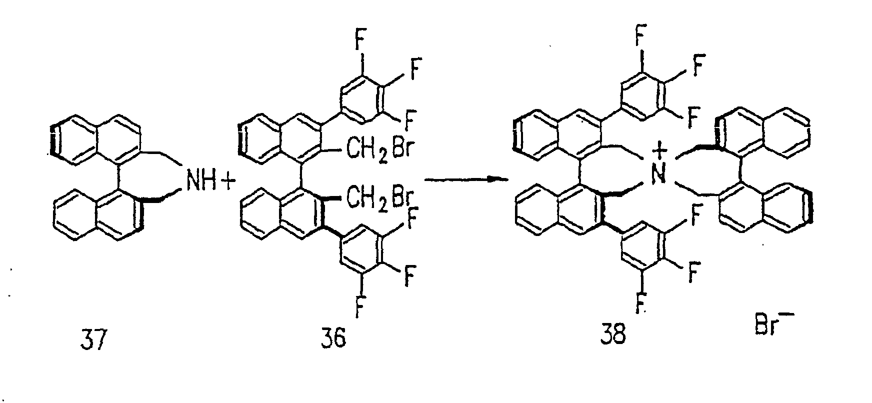

Potassium carbonate (62.5mg, 0.45mmol) was added to a solution of compound (37) (88.6mg, 0.30mmol) in acetonitrile (5mL), and the mixture was stirred at room temperature for 30 minutes. Then, compound (36) (210mg, 0.30mmol) was added. The reaction mixture was stirred with heating at reflux for 3 hours, and then it was poured into water. The mixture was extracted with dichloromethane. The dichloromethane extract was dried over Na₂SO₄, and concentrated under vacuum. The residue was subjected to silica gel chromatography, and elution with methanol - dichloromethane (1:20) gave compound (38) (222mg, 0.24mmol) in 84 % yield.

300MHz ¹H-NMR (CDCl₃): δ 8.27(2H, s, Ar-H), 8.11(2H, d, J=8.4Hz, Ar-H), 7.96(2H, d, J=8.7Hz, Ar-H), 7.65(2H, t, J=7.8Hz, Ar-H), 7.4-7.7(4H, br, Ar-H), 7.52-7.58(4H, m, Ar-H), 7.35(2H, t, J=7.8Hz, Ar-H), 7.24-7.29(2H, m, Ar-H), 7.09-7.15(4H, m, Ar-H), 6.53(2H, d, J=8.4Hz, Ar-H), 4.82(2H, d, J=14.1Hz, ArCH₂), 4.62(2H, d, J=14.1Hz, ArCH₂), 4.46(2H, d, J=13.2Hz, ArCH₂), 3.74(2H, d, J=13.2Hz, ArCH₂) ppm; IR (KBr): ν 3647, 3360, 3055, 2981, 2954, 1614, 1526, 1450, 1360, 1242, 1047, 854, 750cm⁻¹; [α]ₚ²³ +33.6° (CO_{d}2, CHCl₃); MS: m/z 834 (M⁺) (100%), 281, 154, 136, 89.

### Example 37

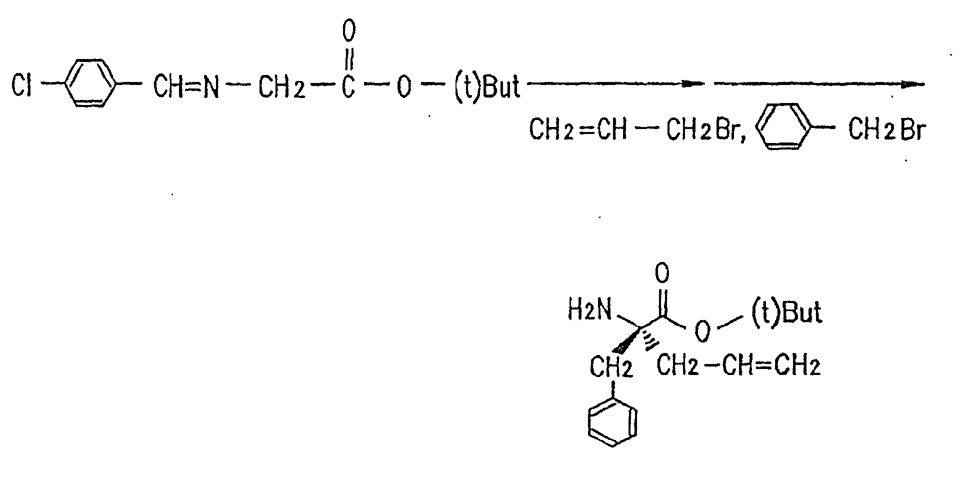

Cesium hydroxide · monohydrate (420mg, 2.5mmol) was added to a mixture of tert-butyl glycinato p-chlorobenzaldehyde Schiff base (127mg, 0.5mmol), a chiral catalyst [compound (38) obtained from Example 36; 4.6mg, 0.005mmol], allyl bromide (43.3 µL, 0.5mmol) in toluene (2mL) at -10°C, and the mixture was stirred for 3.5 hours. After benzyl bromide (72.8µL, 0.6mmol) was added, the mixture was allowed to warm to 0°C and stirred for 30 minutes. Water was added to the mixture, and the mixture was extracted with dichloromethane. After the solvent was evaporated off, the resultant residue was dissolved in tetrahydrofuran (5mL). Then, 0.5M citric acid aqueous solution (5mL) was added, and the mixture was stirred at room temperature for an hour. The aqueous phase was separated, and washed with ether. Sodium bicarbonate was added to the aqueous phase until it became alkaline. Then, the aqueous phase was extracted with dichloromethane. The organic layer was dried over sodium sulfate and concentrated to give an oily residue. This was subjected to silica gel chromatography (eluate, ethyl acetate:hexane = 1:2) to give an alkylated compound (tert-butyl ester of allyl phenylalanine) as a colorless oil: weight, 105mg; yield, 80%; optical purity, 97% (R) [determined by chiral HPLC (DAICEL CHIRALPAK AD; hexane:isoproryl alcohol = 100:1; flow rate 0.5mL/min.); retention time: 14.9 minutes (R), 20.2 minutes (S)].

300MHz ¹H-NMR (CDCl₃): δ 7.21-7.32(5H, m, Ph), 5.65-5.79(1H, m, CH=C), 5.13-5.22(2H, m, C=CH₂), 3.17(1H, d, J=13.2Hz, CHPh), 2.76(1H, d, J=13.2Hz, CHPh), 2.69(1H, dd, J=6.3, 13.5Hz, CHC=C), 2.28(1H, dd, J=8.6, 13.5Hz, CHC=C), 1.60(2H, br s, NH₂), 1.46(9H, s, ^{t}Bu) ppm.

### Example 38

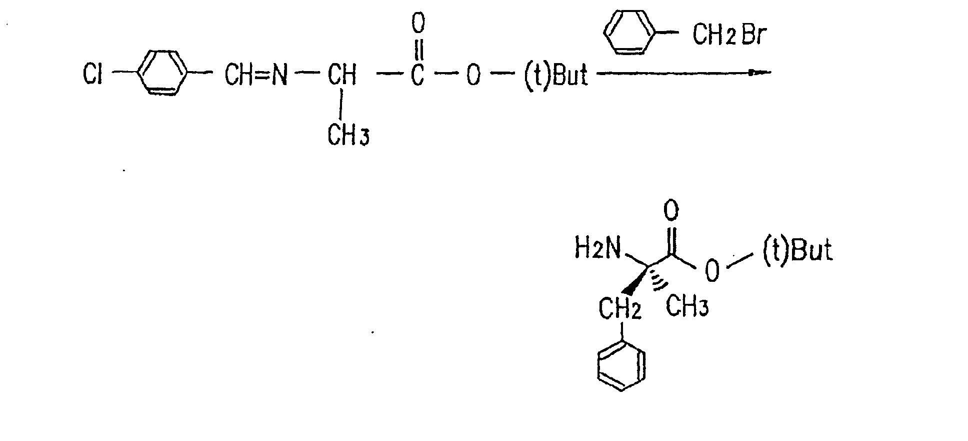

Cesium hydroxide·monohydrate (420mg, 2.5mmol) was added to a mixture of alanine tert-butyl ester p-chlorobenzyl Schiff base (134mg, 0.5mmol), a chiral catalyst (the compound (38) obtained from Example 36; 4.6mg, 0.005mmol), benzyl bromide (72.8 µL, 0.6mmol) and toluene (2mL) at 0°C, and the mixture was stirred at 0°C for 30 minutes. After water was added, the mixture was extracted with dichloromethane. The solvent was evaporated off, and the residue was dissolved in tetrahydrofuran (5mL). Then, 0.5M citric acid aqueous solution (5mL) was added to the solution, and the mixture was stirred at room temperature for an hour. The aqueous phase was washed with ether. Sodium bicarbonate was added to the aqueous phase until it became alkaline. Then, the aqueous phase was extracted with dichloromethane. The organic layer was dried over sodium sulfate, and concentrated to obtain an oily product. The oily product thus obtained was subjected to silica gel chromatography (eluate; ethyl acetate:hexane = 2:1) to give an alkylated product (benzylalanine t-butylester) as a colorless oil; weight, 100mg; yield, 85%; optical purity, 98% (R) [determined by chiral HPLC (DAICEL CHIRALPAK AD; hexane:isopropyl alcohol = 30:1, flow rate 0.5mL/min.); retention time: 12.9 minutes (R), 20.5 minutes (S)].

300MHz ¹H-NMR (CDCl₃): δ 7.20-7.30(5H, m, Ph), 3.12(1H, d, J=13.2Hz, CHPh), 2.78(1H, d, J=13.2Hz, CHPh), 1.64(2H, s, NH₂), 1.46(9H, s, ^{t}Bu), 1.35(3H, s, CH₃) ppm.

### Examples 39-42

The following alkylated products were synthesized in the same manner as in Example 38.

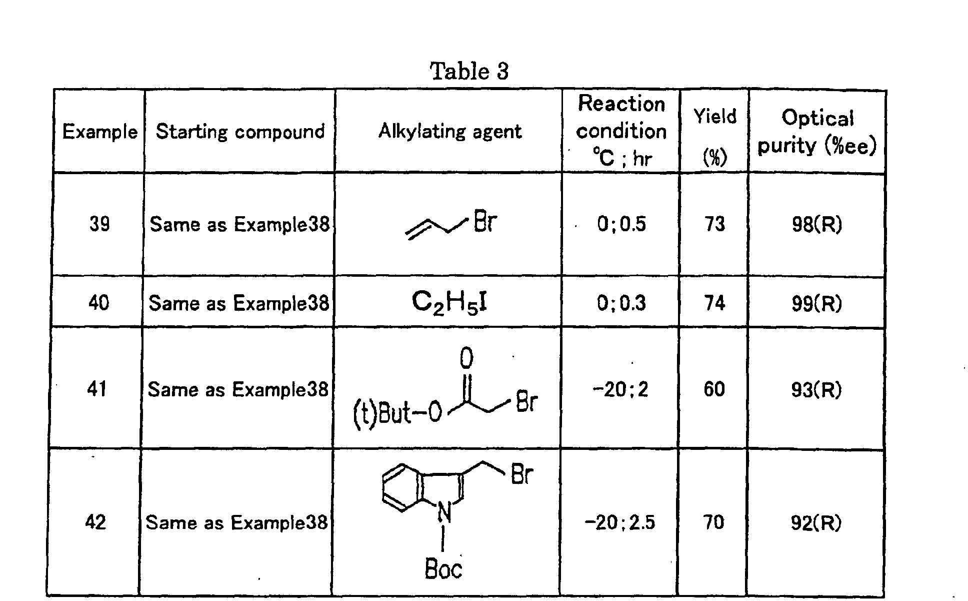

NMR spectra and analytical conditions of HPLC regarding the resultant alkylated products are shown as follows.

### Example 39

300MHz ¹H-NMR (CDCl₃): δ 5.65-5.80(1H, m, CH=C), 5.11-5.17(2H, m, C=CH₂), 2.50(1H, dd, J=6.6, 13.5Hz, CHC=C), 2.23(1H, dd, J=8.3, 13.5Hz, CHC=C), 1.60(2H, s, NH₂), 1.46(9H, s, ^{t}Bu), 1.29(3H, s, CH₃); HPLC analysis of the corresponding N-benzoate: DAICEL CHIRALCEL OD, hexane:isopropyl alcohol = 100:1, flow rate = 0.5mL/min; retention time, 17.6min(R) and 25.9min(S).

### Example 40

300MHz ¹H-NMR (CDCl₃): δ 1.66-1.79(1H, m, CHCH₃), 1.65(2H, br, NH₂), 1.48-1.60(1H, m, CHCH₃), 1.46(9H, s, ^{t}Bu), 1.27(3H, s, CH₃), 0.87(3H, t, J=7.5Hz, CH₂CH₃); HPLC analysis of the corresponding N-benzoate: DAICEL CHIRALPAK AD, hexane:isopropyl alcohol = 150:1, flow rate = 0.5mL/min; retention time, 28.1min(R) and 31.5min(S).

### Example 41

300MHz ¹H-NMR (CDCl₃): δ 2.82(1H, d, J=16.8Hz, CHC=O), 2.44(1H, d, J=16.8Hz, CHC=O), 1.86(2H, br s, NH₂), 1.45(9H, s, ^{t}Bu), 1.44(9H, s, ^{t}Bu), 1.26(3H, s, CH₃); HPLC analysis of the corresponding N-benzoate: DAICEL CHIRALCEL OD, hexane:isopropyl alcohol = 100:1, flow rate = 0.5mb/min; retention time, 14.9min(R) and 20.6min(S).

### Example 42

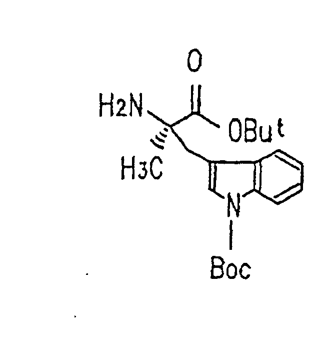

300MHz ¹H-NMR (CDCl₃): δ 8.15(1H, m, Ph), 7.64(1H, m, Ph), 7.45(1H, br s, C=CH-N), 7.22-7.34(2H, m, Ph), 3.18(1H, dd, J=0.9, 14.1Hz, CHC=C-N), 2.93(1H, dd, J=0.6, 14.1Hz, CHC=C-N), 1.65(9H, s, ^{t}Bu), 1.62(2H, s, NH₂), 1.46(9H, s, ^{t}Bu), 1.41(3H, s, CH₃); HPLC analysis: DAICEL CHIRALPAK AD, hexane:isopropyl alcohol = 30:1, flow rate = 0.5mL/min; retention time, 12.2min(R) and 17.7min(S).

### Example 43

Benzylated compounds were obtained by using compound produced in Reference Example 1 in the same manner as in Example 38 (yield 75%, optical purity 87%). Moreover, the allylation product was also obtained in the same manner [yield, 72%; optical purity, 97%e.e.(R)]. The characteristics of those compounds are shown as follows.

### benzylation product

300MHz ¹H-NMR (CDCl₃): δ 7.20-7.30(5H, m, Ph), 3.12(1H, d, J=13.1Hz, PhCH), 2.71(1H, d, J=13.1Hz, PhCH), 1.88(1H, dd, J=6.8, 13.7Hz, ⁱPrCH), 1.75(1H, m, (CH₃)₂CH), 1.56(2H, br s, NH₂), 1.53(1H, dd, J=5.3, 13.7Hz, ⁱPrCH), 1.44(9H, s, ^{t}Bu), 0.98(3H, d, J=6.8Hz, CH₃), 0.91(3H, d, J=6.8Hz, CH₃). IR (liquid film): 2957, 1724, 1603, 1497, 1456, 1393, 1367, 1236, 1153, 1128, 849, 739, 702cm⁻¹. MS: m/z278([M+H]⁺), 186(100%), 176, 91, 57. HRMS C₁₇H₂₇NO₂ (calculated): 278.2120([M+H]⁺). (observed): 278.2135([M+H]⁺). HPLC analysis: DAICEL CHIRALPAK AD, hexane:isopropyl alcohol = 100:1, flow rate = 0.5mL/min, retention time; 13.9min(majar enantiomer) and 15.6min(minor enantiomer).

### allylation product

300MHz ¹H-NMR (CDCl₃): δ 5.61-5.75(1H, m, CH=C), 5.11-5.17(2H, m, C=CH₂), 2.52(1H, ddt, J=1.2, 6.3, 13.5Hz, CHC=C), 2.16(1H, dd, J=8.6, 13.5Hz, CHC=C), 1.68-1.79(2H, m, (CH₃)₂CH and ⁱPrCH), 1.59(2H, br s, NH₂), 1.44-1.54(1H, m, ⁱPrCH), 1.47(9H, s, ^{t}Bu), 0.95(3H, d, J=6.5Hz, CH₃), 0.88(3H, d, J=6.5Hz, CH₃). IR (liquid film) 3080, 2957, 2918, 1726, 1641, 1603, 1474, 1393, 1367, 1234, 1144, 993, 920, 853, 756, 664cm⁻¹. MS: m/z228([M+H)⁺)(100%), 226, 186, 170, 85, 57, 37. HRMS C₁₃H₂₅NO₂ (calculated): 228.1963([M+H]⁺). (observed): 228.1948 ([M+H]⁺). HPLC analysis: DAICEL CHIRALCEL OD, hexane:isopropyl alcohol = 150:1, flow rate = 0.5mL/min, retention time; 12.4min(minor enantiomer) and 15.8min(major enantiomer).

### Example 44

In the same manner as in Example 37, dialkyl compounds were produced by using 2-methyl-2-propenyl bromide or propargyl bromide instead of benzyl bromide. The characteristics of the compounds are shown as follows.

### 2-methyl-2-propenyl product

300MHz ¹H-NMR (CDCl₃): δ 5.64-5.78(1H, m, CH=C), 5.12-5.19(2H, m, C=CH₂), 4.90(1H, m, C(CH₃)=CH), 4.80(1H, m, C(CH₃)=CH), 2.62(1H, d, J=14.0Hz, CHC(CH₃)=C), 2.56(1H, ddt, J=1.2, 6.6, 13.5Hz, CHC=C), 2.24(1H, d, J=14.0Hz, CHC(CH₃)=C), 2.20(1H, dd, J=8.3, 13.5Hz, CHC=C), 1.74(3H, s, CH₃C=C), 1.64(2H, s, NH₂), 1.43(9H, s, ^{t}Bu). IR (liquid film) 3078, 2978, 2924, 1726, 1641, 1597, 1458, 1393, 1369, 1229, 1159, 1053, 993, 899, 843cm⁻¹. MS: m/z226([M+H]⁺)(100%), 184, 170, 124, 57. HRMS C₁₃H₂₃NO₂ (calculated): 226.1806([M+H]⁺). (observed): 226.1795([M+H]⁺). HPLC analysis: DAICEL CHIRALCEL OD, hexane:isopropyl alcohol = 300:1, flow rate = 0.5mL/min, retention time; 25.3min(major enantiomer) and 35.1min(minor enantiomer).

### propargyl product

300MHz ¹H-NMR (CDCl₃): δ 5.65-5.79(1H, m, CH=C), 5.12-5.20(2H, m, C=CH₂), 2.64(1H, dd, J=2.7, 16.5Hz, CHC≡C), 2.52(1H, ddt, J=1.2, 6.9, 13.5Hz, CHC=C), 2.41(1H, dd, J=2.7, 16.5Hz, CHC≡C), 2.29(1H, ddt, J=0.9, 8.0, 13.5Hz, CHC=C), 2.05(1H, t, J=2.7Hz, C≡CH), 1.75(2H, br s, NH₂), 1.48(9H, s, ^{t}Bu). IR (liquid film) 3377, 3310, 3078, 2980, 2932, 1732, 1641, 1597, 1437, 1394, 1369, 1329, 1231, 1159, 1034, 997, 920, 845, 752, 646cm⁻¹. MS: m/z210([M+H]⁺)(100%), 168, 108, 57. HRMS C₁₂H₁₉NO₂ (calculated): 210.1494([M+H]⁺). (observed): 210.1485([M+H]⁺). GC analysis: GL SCIENCE CP-CHIRASIL-DEX CS, retention time; 16.1min(minor enantiomer) and 16.7min(major enantiomer).

### Reference Example 1

Aldimine Schiff base was prepared by reacting leucine tert-butyl ester as a raw material with p-chlorobenzaldehyde according to the conventional procedures. The characteristics of the Schiff base are shown as follows.

300MHz ¹H-NMR (CDCl₃): δ 8.24(1H, s, CH=N), 7.73(2H, d, J=8.7Hz, p-Cl-Ph), 7.39(2H, d, J=8.7Hz, p-Cl-Ph), 3.96(1H, dd, J=6.2, 8.0Hz, CHC=O), 1.77-1.82(2H, m, ⁱPrCH₂), 1.56(1H, m, (CH₃)₂CH), 1.47(9H, s, ^{t}Bu), 0.95(3H, d, J=6.6Hz, CH₃), 0.89(3H, d, J=6.6Hz, CH₃). IR (KBr) 2980, 2959, 2934, 1736, 1641, 1597, 1573, 1491, 1466, 1393, 1366, 1339, 1275, 1209, 1146, 1088, 1063, 1015, 829, 772cm⁻¹. MS: m/z310([M+H]⁺), 308, 210, 208, 57(100%). C₁₇H₂₄ClNO₂ (calculated): C, 65.90; H, 7.81; N, 4.52; Cl, 11.44. (observed): C, 65.92; H, 7.84; N, 4.55; Cl, 11.39.

### Reference Example 2

Aldimine Schiff base was prepared by reacting phenylalanine tert-butyl ester as a raw material with p-chlorobenzaldehyde according to the conventional procedures. The characteristics of the Schiff base are shown as follows:

300MHz ¹H-NMR (CDCl₃): δ 7.88(1H, s, CH=N), 7.64(2H, d, J=8.7Hz, p-Cl-Ph), 7.36(2H, d, J=8.7Hz, p-Cl-Ph), 7.16-7.24(5H, m, Ph), 4.06(1H, dd, J=5.4, 8.7Hz, CHC=O), 3.32(1H, dd, J=5.4, 13.5Hz, PhCH), 3.10(1H, dd, J=8.7, 13.5Hz, PhCH), 1.44(9H, s, ^{t}Bu). IR (KBr) 2984, 2882, 2808, 1724, 1647, 1593, 1491, 1373, 1279, 1155, 1084, 847, 826, 760, 700cm⁻¹, MS: m/z343(M⁺)(100%), 278, 244, 242, 186, 91, 57, C₂₀H₂₂ClNO₂ (calculated): C, 69.86; H, 6.45; N, 4.07; Cl, 10.31. (observed): C, 69.89; H, 6.57; N, 4.05; Cl, 10.33.

### Industrial Applicability

According to the present invention, a novel optically active quarternary ammonium salt with axial chirality is provided. The quarternary ammonium salt of the present invention is used as a phase-transfer catalyst in order to convert glycine derivatives into optically active α-amino acid derivatives by stereoselectively alkylating the glycine derivatives. Moreover, according to the present invention, an intermediate useful for production of the novel quarternary ammonium salt is provided.

## Claims

1. A compound of the formula (Ia): 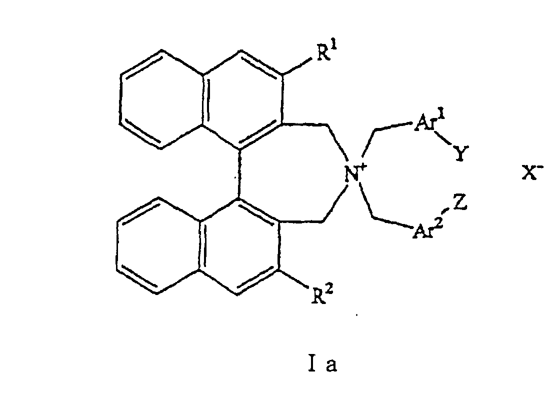 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different;
X⁻ is a halide anion; and
Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different, or may form a single bond,
with the proviso that the case where both R¹ and R² are a hydrogen atom and both Ar¹-Y and Ar²-Z are an unsubstituted phenyl group or an unsubstituted α-naphthyl group, and the case where both R¹ and R² are a hydrogen atom, an unsubstituted phenyl group or an unsubstituted β-naphthyl group and Ar¹-Y and Ar²-Z form a group represented by the following formula: 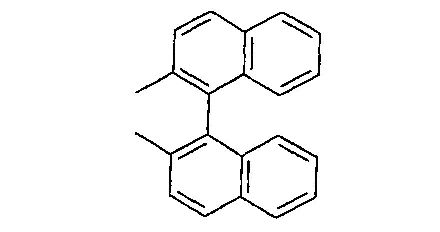 are excluded.

2. The compound of Claim 1, wherein the compound is a spiro type, and Y and Z form a single bond, and the compound is represented by the formula (IIa):

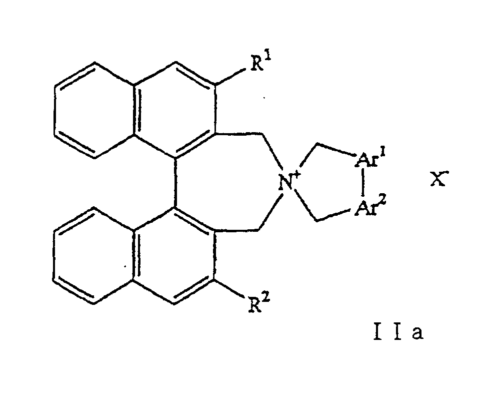

3. A method for producing the compound of Claim 1, comprising reacting a compound of the formula (IVa): 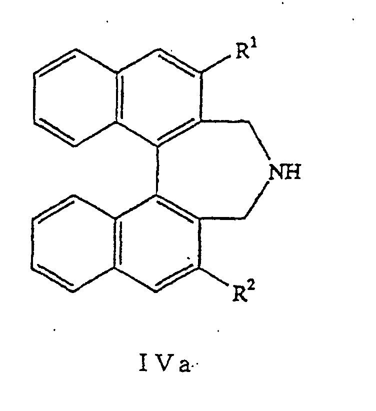 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different, with the proviso that the case where both R¹ and R² are a hydrogen atom is excluded; with a compound of the formula (V): 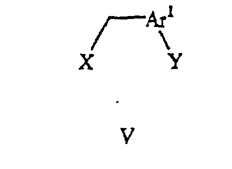 and a compound of the formula (VI): 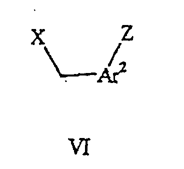 wherein, in the formulae (V) and (VI), Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different; X is a halogen atom; and Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond, with the proviso that the case where both Ar¹-Y and Ar²-Z are an unsubstituted phenyl group or an unsubstituted α-naphthyl group is excluded;
in this order or simultaneously, in the presence of an acid scavenging agent and in an appropriate solvent,

4. A method for producing the compound of Claim 2, comprising reacting a compound of the formula (IVa): 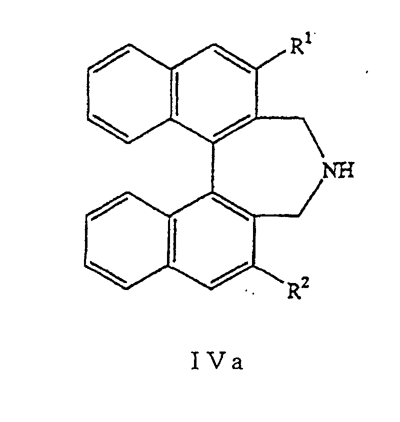 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
with a compound of the formula (VIIa): 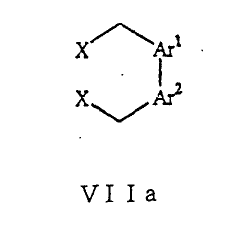 wherein Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different; X is a halogen atom;
in the presence of an acid scavenging agent and in an appropriate solvent,
with the proviso that the case where both R¹ and R² are a hydrogen atom and 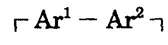 is an unsubstituted phenyl group or a binaphthyl group which is bound to an unsubstituted α-naphthyl group are excluded.

5. A compound of the formula (VIIIa): 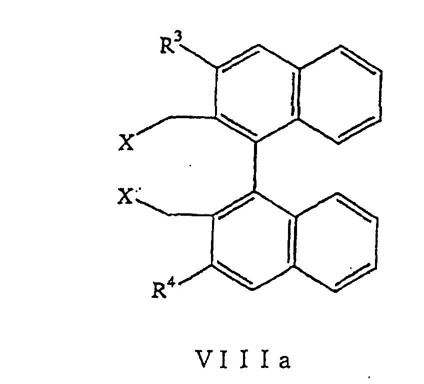 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R³ and R⁴ may be the same or different; and X is a halogen atom, with the proviso that the case where both R³ and R⁴ are a hydrogen atom, an unsubstituted phenyl group, or an unsubstituted β -naphthyl group is excluded.

6. The compound of Claim 5, wherein X is a bromine atom.

7. A compound of the formula (XIa): 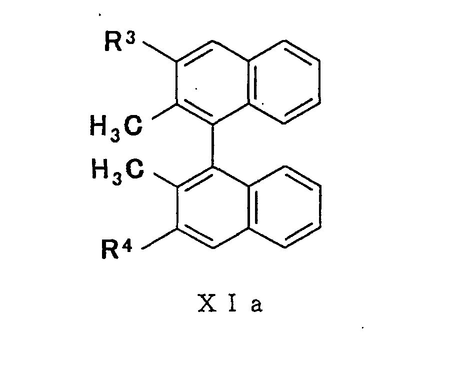 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R³ and R⁴ may be the same or different, with the proviso that the case where both R³ and R⁴ are a hydrogen atom is excluded.

8. A method for producing the compound of Claim 5, comprising reacting the compound of Claim 7 with an appropriate halogenating agent which can generate a halogen radical in an appropriate solvent and in the presence of a radical reaction initiator to halogenate both 2-and 2'-methyl groups.

9. A compound of the formula (XIIa): 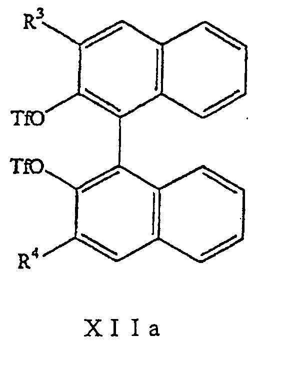 wherein R³ and R⁴ are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₃ alkyl) carbamoyl group; and a N,N-di(C₁ to C₃ alkyl) carbamoyl group (wherein alkyl may be the same or different), and R³ and R⁴ may be the same or different; and Tf is a trifluoromethanesulfonyl group, with the proviso that the case where both R³ and R⁴ are an unsubstituted phenyl group or an unsubstituted β-naphthyl group is excluded.

10. A method for producing the compound of Claim 7, comprising reacting the compound of Claim 9 with a methylmagnesium halide represented by the formula (XIII):
MeMgX (XIII)
wherein X is a halogen atom,
in the presence of a nickel catalyst and in an appropriate solvent.

11. A method for producing a compound of the formula (XIIa'): 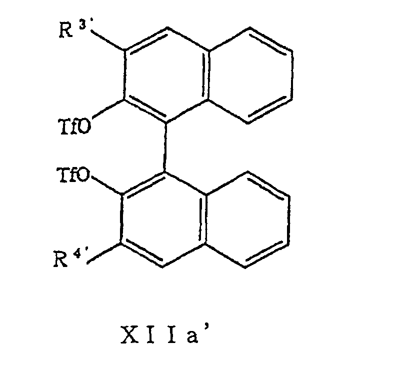 wherein R^{3'} and R^{4'} are groups independently selected from the group consisting of an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₃ alkyl) carbamoyl group; and a N,N-di(C₁ to C₃ alkyl) carbamoyl group (wherein alkyl may be the same or different), and R^{3'} and R^{4'} may be the same or different; and Tf is a trifluoromethanesulfonyl group, with the proviso that the case where both R³ and R⁴ are an unsubstituted phenyl group or an unsubstituted β-naphthyl group is excluded, comprising:
substituting a bromine atom in a compound of the formula (XIV): 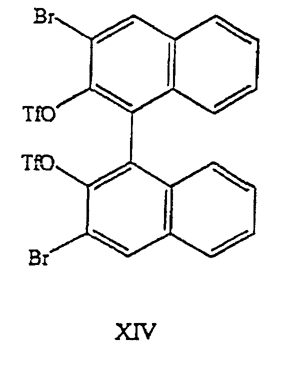 wherein Tf is a trifluoromethanesulfonyl group,
with R^{3'} and R^{4'} in an appropriate solvent and in the presence of a transition metal catalyst.

12. A method for producing a compound of the formula (XVIIa): 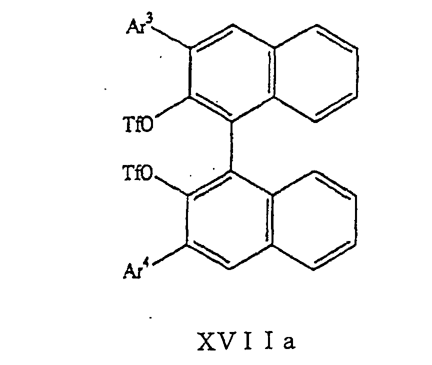 wherein Ar³ and Ar⁴ are groups selected from the group consisting of an alkenyl group having 2 to 6 carbon atoms which may be branched or form a cyclic group; an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar³ and Ar⁴ may be the same or different, with the proviso that the case where both Ar³ and Ar⁴ are an unsubstituted phenyl group or an unsubstituted β-naphthyl group is excluded; comprising
reacting a compound of the formula (XIV): 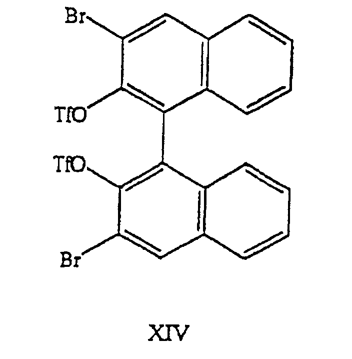 wherein Tf is a trifluoromethanesulfonyl group,
with a compound of the formula (XV):
Ar³B(OH)₂ (XV)
and a compound of the formula (XVI):
Ar⁴B(OH)₂ (XVI)
wherein, in the formulae (XV) and (XVI), Ar³ and Ar⁴ are groups selected from the group consisting of an alkenyl group having 2 to 6 carbon atoms which may be branched or form a cyclic group; an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms, or a halogen atom, and Ar³ and Ar⁴ may be the same or different; in this order or simultaneously, in an appropriate solvent and in the presence of a base and a palladium catalyst.

13. A compound of the formula (XVIII):

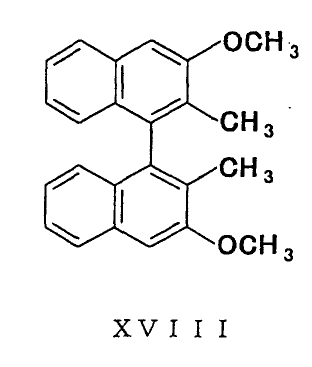

14. A method for producing a compound of the formula (XVIII): 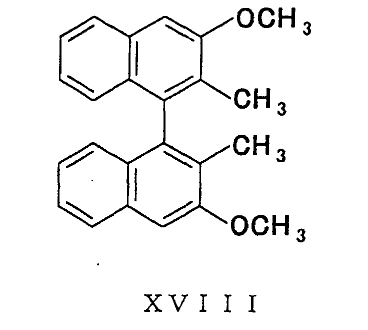 comprising:
sulfonating a compound of the formula (XXI): 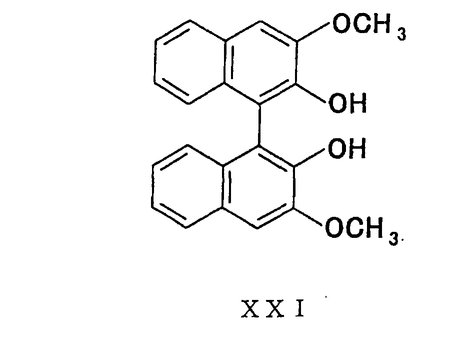 to generate a compound of the formula (XXII): 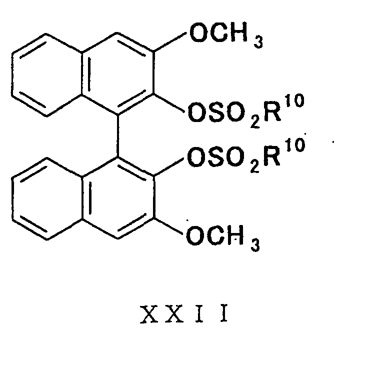 wherein R¹⁰ is an alkyl group having 1 to 4 carbon atoms which may be substituted with a halogen atom; and
reacting the compound of the formula (XXII) with a methylmagnesium halide of the formula (XIII):
MeMgX (XIII)
wherein X is a halogen atom, in an appropriate solvent and in the presence of a nickel catalyst.

15. A compound of the formula (XXIII):

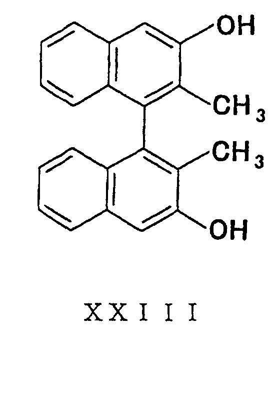

16. A method for producing a compound of the formula (XXIII): 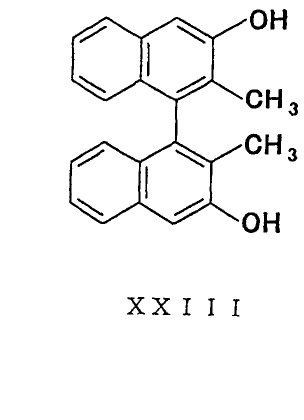 comprising:
reacting a compound of the formula (XVIII): 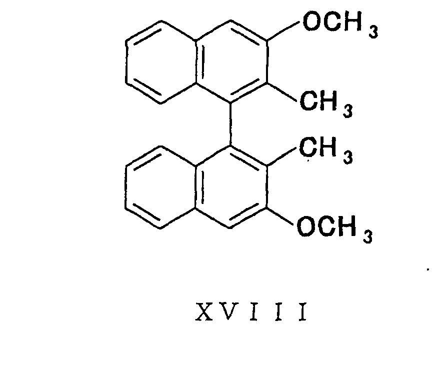 with boron tribromide.

17. A compound of the formula (XXIV):

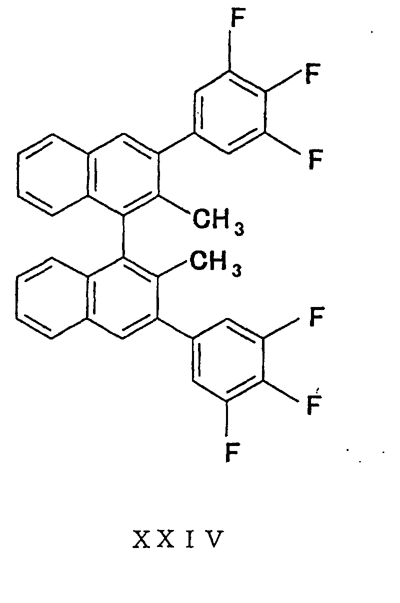

18. A method for producing a compound of the formula (XXIV): 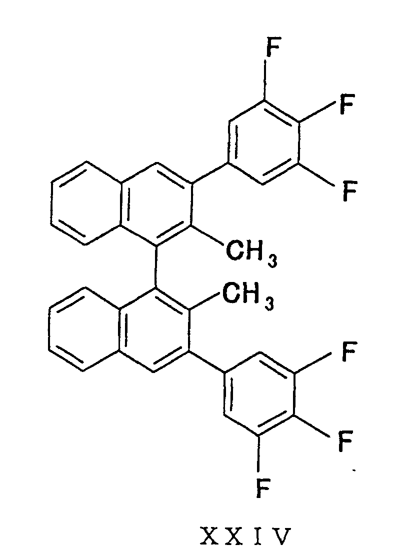 comprising:
sulfonating a compound of the formula (XXIII): 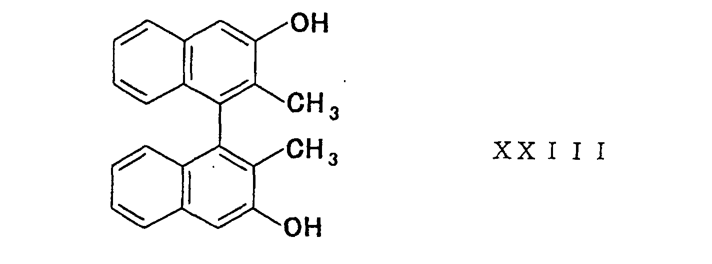 to generate a compound of the formula (XXV): 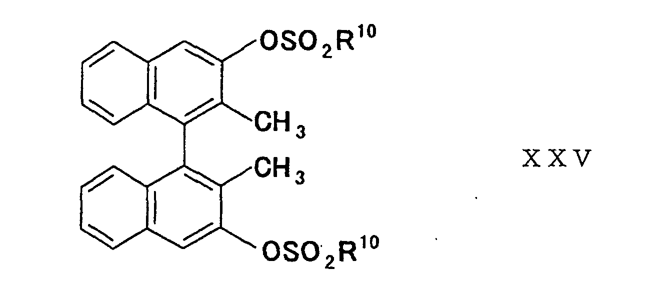 wherein R¹⁰ is an alkyl group having 1 to 4 carbon atoms which may be substituted with a halogen atom; and
reacting the compound of the formula (XXV) with 3,4,5-trifluorophenyl boronic acid, in an appropriate solvent and in the presence of a palladium catalyst.

19. A compound of the formula (XXVI):

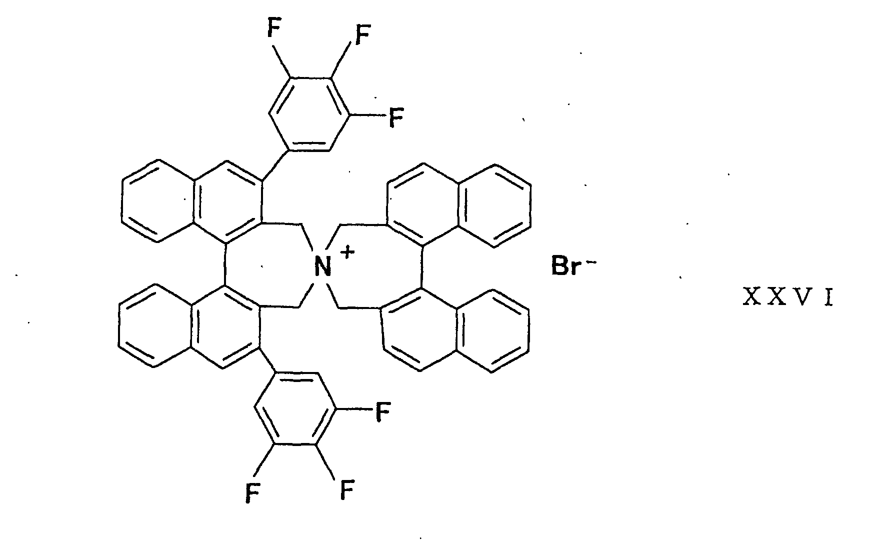

20. A method for producing a compound of the formula (XXVI): 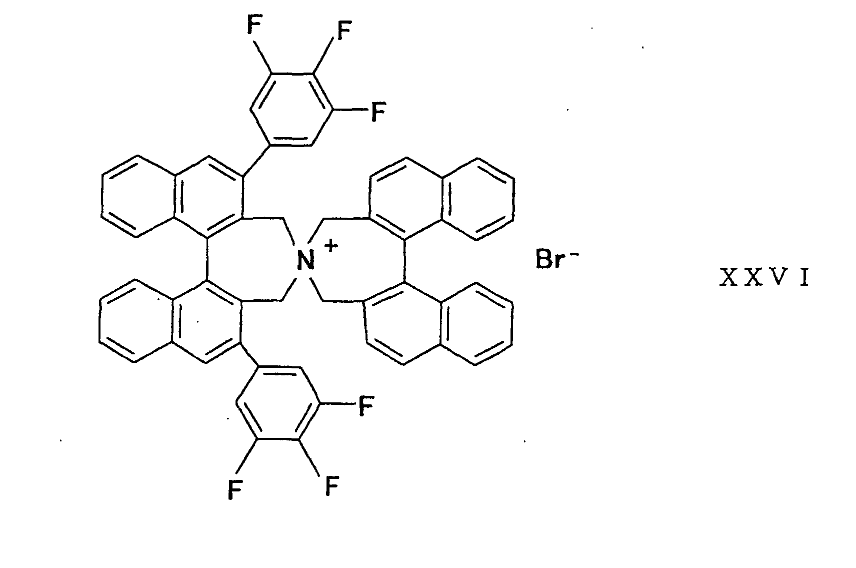 comprising:
reacting a compound of the formula (XXIV): 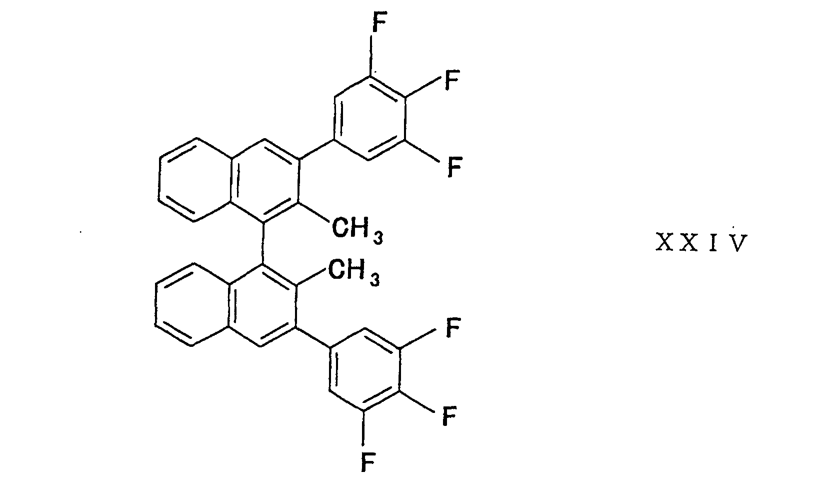 with bromosuccinimide and 2,2'-azobis(isobutylonitrile), in an appropriate solvent to generate a compound of the formula (XXVII): 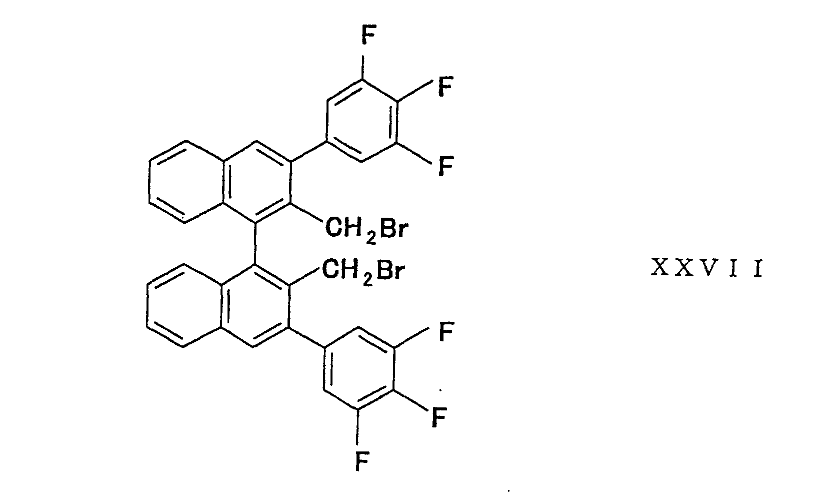 and
reacting the compound of the formula (XXVII) with a compound of the formula (XXVIII):

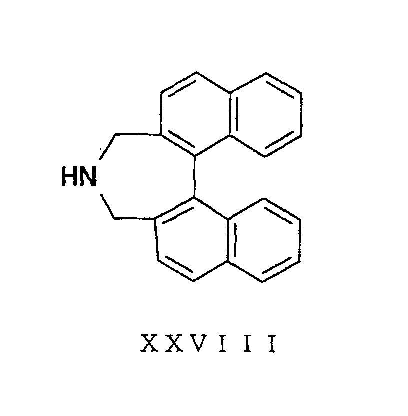

21. A method for stereoselectively producing a compound of the formula (XXIa): 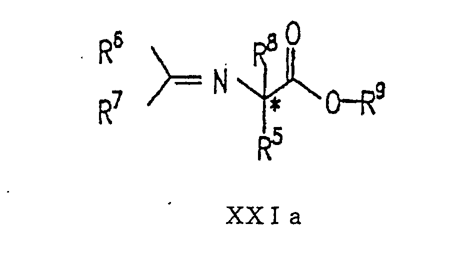 wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which having 3 to 9 carbon atoms which may be branched;
R⁶ and R⁷ are the same or different and are hydrogen atoms, an aryl group which may be substituted with an alkyl group having 1 to 3 carbon atoms, an alkoxy group having 1 to 3 carbon atoms or a halogen atom, with proviso excepting that they are hydrogen atoms simultaneously;
R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, an alkoxy group having 1 to 3 carbon atoms or a halogen atom; R⁹ is an alkyl group having 1 to 4 carbon atoms; and
* is a newly produced chiral center,
comprising;
alkylating a compound represented by the formula (XIXa): 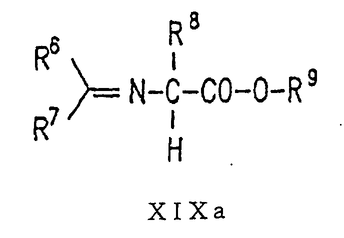 wherein R⁶ and R⁷ are the same or different and are hydrogen atoms, an aryl group which may be substituted with an alkyl group having 1 to 3 carbon atoms, an alkoxy group having 1 to 3 carbon atoms or a halogen atom, with proviso excepting that they are hydrogen atoms simultaneously; R⁸ is a hydrogen atom; an aryl group which may be substituted with an alkyl group having 1 to 3 carbon atoms, an alkoxy group having 1 to 3 carbon atoms or a halogen atom; an alkyl group having 1 to 6 carbon atoms which may be branched or form a cyclic group; an aralkyl group which may be substituted with an alkyl group having 1 to 3 carbon atoms, an alkoxy group having 1 to 3 carbon atoms or a halogen atom; and R⁹ is an alkyl group having 1 to 4 carbon atoms; with a compound of the formula (XX):
R⁵-W (XX)
wherein R⁵ is an alkyl group having 1 to 6 carbon atoms which may be branched or form a cyclic group; an allyl or substituted allyl group having 3 to 9 carbon atoms which may be branched or form a cyclic group; an aralkyl group which may be substituted with an alkyl group having 1 to 4 carbon atoms, an alkoxy group having 1 to 3 carbon atoms, a halogen atom, an aryl group which may be substituted with an alkyl group having 1 to 4 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; a heteroaralkyl group which may be substituted with an alkyl group having 1 to 3 carbon atoms, an alkoxy group having 1 to 3 carbon atoms, a halogen atom, an aryl group which may be substituted with an alkyl group having 1 to 3 carbon atoms, an alkoxy group having 1 to 3 carbon atoms or a halogen atom, or a heteroaryl group which may be substituted with an alkyl group having 1 to 4 carbon atoms, an alkoxy group having 1 to 3 carbon atoms or a halogen atom; or a propargyl or substituted propargyl group which having 3 to 9 carbon atoms which may be branched; and W is a functional group having leaving ability, using a compound of the formula (I), 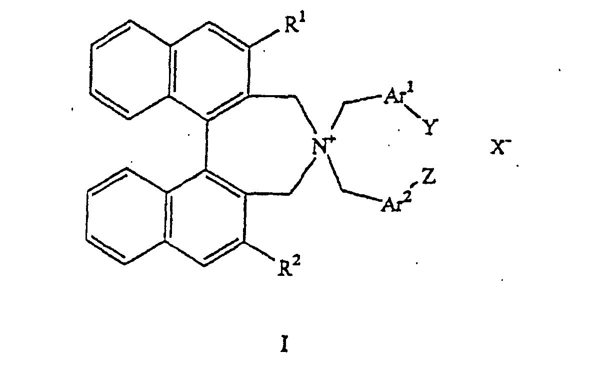 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein an alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different;
X⁻ is a halide anion; and
Y and Z are groups independently selected from the group consisting of a hydrogen atom; a halogen atom; an alkyl group having 1 to 4 carbon atoms; and an alkoxy group having 1 to 3 carbon atoms, and Y and Z may be the same or different or may form a single bond;
which is pure regarding axial chirality, as a phase-transfer catalyst in a solvent and in the presence of an inorganic base,
with the proviso that the case where, in the compound of the formula (I), both R¹ and R² are a hydrogen atom and both Ar¹-Y and Ar²-Z are an unsubstituted phenyl group or an unsubstituted α-naphthyl group, and, in the compound of the formula (XX), R⁵ is a benzyl group; the case where, in the compound of the formula (I), both R¹ and R² are an unsubstituted β-naphthyl group and Ar¹-Y and Ar²-Z form a group represented by the following formula: 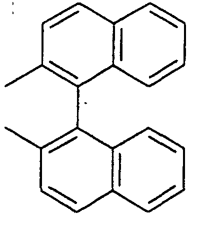 and, in the compound of the formula (XX), R⁵ is a benzyl group; and the case where, in the compound of the formula (I), both R¹ and R² are an unsubstituted β -naphthyl group and, in the compound of the formula (XX), R⁵ is a benzyl group, a methyl group, an ethyl group, an allyl group, a 2-methylpropene group, a propargyl group, a p-methylbenzyl group, p-fluorobenzyl group, or a group represented by the following formula: 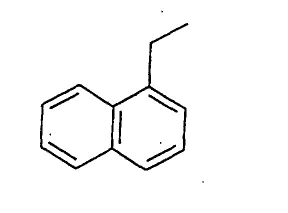 are excluded.

22. The method of Claim 21, wherein the phase-transfer catalyst is the compound of the formula (II): 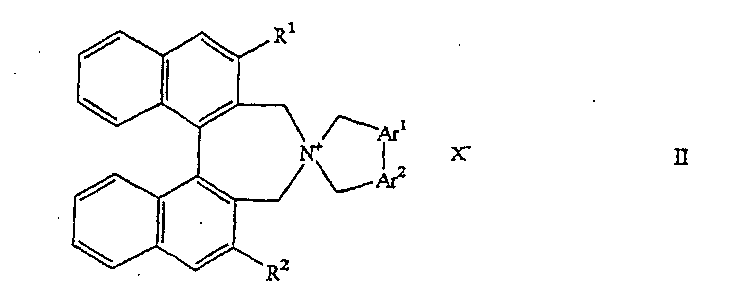 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
Ar¹ and Ar² are groups independently selected from the group consisting of an aryl group which may be substituted with an alkyl group having 1 to 4 carbon atoms, an alkoxy group having 1 to 3 carbon atoms or a halogen atom; and a heteroaryl group which may be substituted with an alkyl group having 1 to 4 carbon atoms, an alkoxy group having 1 to 3 carbon atoms or a halogen atom, and Ar¹ and Ar² may be the same or different;
X⁻ is a halide anion; and
Y and Z are groups independently selected from the group consisting of a hydrogen atom; a halogen atom; an alkyl group having 1 to 4 carbon atoms; and an alkoxy group having 1 to 3 carbon atoms;
with the proviso that the case where both R¹ and R² are a hydrogen atom, an unsubstituted phenyl group, or an unsubstituted β -naphthyl group, and both Ar¹-Y and Ar²-Z form a group represented by the following formula: 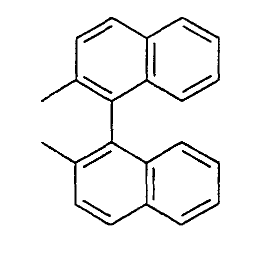 is excluded.

23. The method of Claim 21, wherein the phase-transfer catalyst is the compound of the formula (III): 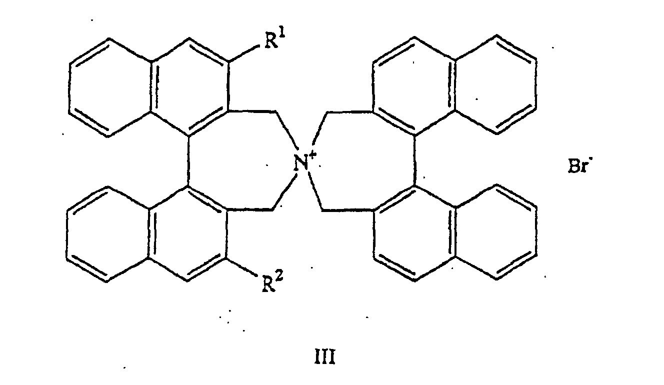 wherein R¹ and R² are groups independently selected from the group consisting of a hydrogen atom; an alkyl group having 1 to 6 carbon atoms which may be branched or form a cyclic group; an alkenyl group having 2 to 6 carbon atoms which may be branched or form a cyclic group; an alkynyl group having 2 to 6 carbon atoms which may contain branched or cyclic structure; an aralkyl group which may be substituted with an alkyl group having 1 to 4 carbon atoms, an alkoxy group having 1 to 3 carbon atoms or a halogen atom; a heteroaralkyl group which may be substituted with an alkyl group having 1 to 4 carbon atoms, an alkoxy group having 1 to 3 carbon atoms or a halogen atom; an aryl group which may be substituted with an alkyl group having 1 to 4 carbon atoms, an alkoxy group having 1 to 3 carbon atoms or a halogen atom; a heteroaryl group which may be substituted with an alkyl group having 1 to 4 carbon atoms, an alkoxy group having 1 to 3 carbon atoms or a halogen atom; a (C₁ to C₃ alkoxy) carbonyl group; a carbamoyl group; a N-(C₁ to C₄ alkyl) carbamoyl group; and a N,N-di(C₁ to C₄ alkyl) carbamoyl group (wherein alkyl may be the same or different), and R¹ and R² may be the same or different;
with the proviso that the case where both R¹ and R² are a hydrogen atom, an unsubstituted phenyl group, or an unsubstituted β-naphthyl group is excluded.

24. The method of Claim 23, wherein both R¹ and R² in the compound of the formula (III) are the following formula:
